# EUROPEAN PATENT APPLICATION

(11) **EP 4 843 588 A1**
(43) Date of publication of application: **25.11.2026**
(21) Application number: 25742027.3
(22) Date of filing: 17.01.2025
(51) Int. Cl.: A61L 27/38, C12N 5/077

(54) **BONE SUBSTITUTE AND METHOD FOR PRODUCING SAME**

(30) Priority: 19.01.2024 JP 2024007044
(71) Applicant: Tohoku University, Sendai-shi, Miyagi 980-8577 (JP)
(72) Inventor: EGUSA, Hiroshi, Sendai-shi, Miyagi 980-8577 (JP); OKAWA, Hiroko, Sendai-shi, Miyagi 980-8577 (JP); MORI, Satomi, Sendai-shi, Miyagi 980-8577 (JP)
(74) Representative: Schön, Christoph
(86) International application number: PCT/JP2025/001383
(87) International publication number: WO 2025/154802

(57) **Abstract**

Stem cells were induced to differentiate into osteoblasts by three-dimensional culture to form cell aggregates, thereby producing bone substitutes. By employing a freeze-drying method, the risk of tumor formation can be reduced, and immunogenicity and local inflammation can be controlled. By optimizing the temperature and time of an initial freezing step, a primary drying step, and a secondary drying step, it is possible to provide a bone substitute having good performance and reduced cost. It is possible to provide a bone substitute having high safety and having higher osteoconductive ability and osteoinductive ability.

## Description

### Technical Field

The present invention relates to a bone substitute to be used for surgical treatment of a tissue of a damaged bone, and a method for producing the same.

### Background Art

Bone tissues have a plurality of important functions such as protection of the brain and internal organs, support of the skeleton, production of blood, and storage of electrolytes such as calcium. A bone tissue is damaged by a trauma, a surgical resection of a bone tumor, an infectious disease, an osteomyelitis, or the like. When the damage is mild, osteoblasts proliferate and differentiate, and bones are regenerated and cured. However, in the case of a large defect or in the case of an environment in which osteoblasts cannot function effectively, treatment using bone transplantation may be required. With the progress of aging, the number of elderly people with low bone density is increasing, and the markets of bone graft and artificial bone transplant are expanding year by year. Therefore, the development of bone substitutes is progressing.

The bone substitutes are roughly classified into an autologous bone, an allogeneic bone, a xenogeneic bone, and an artificial bone transplant. The autologous bone graft is considered as a golden standard for a large-scale femoral defect, but has disadvantages such as invasion to a donor site, a limited supply amount, and infection. A freeze-dried bone graft and a demineralized freeze-dried bone graft have relatively good osteoinductivity, but a supply amount of the freeze-dried bone graft and the demineralized freeze-dried bone graft is limited, and there is a possibility of a risk of infection with an unknown virus or a bacterium. In addition, it is pointed out that artificial bone substitutes also have the following problems. For example, β-tricalcium phosphate (β-TCP) is rapidly absorbed in a living body, so that a sufficient amount of bone cannot be secured. In contrast, hydroxyapatite and carbonate apatite are not easily absorbed and are useful for securing a space for bone regeneration, but remain for a long period of time, and thus have a risk of increase in infection and vulnerability.

In the dental field, these disadvantages are addressed by combining autologous bones and bone substitutes. However, it is reported that about 25% of all patients who undergo a maxillary sinus floor elevation surgery using a combination of an autologous bone and an artificial bone experience a bone resorption one year after the surgery, and it is also reported that a bone height decreases by 25% six months after an autologous bone graft by an onlay graft method. Therefore, it is pointed out that bone regeneration is not sufficiently achieved. Therefore, development of a new bone substitute capable of creating a minute environment that promotes bone remodeling is awaited.

The induced pluripotent stem cell (iPSC) can be produced by introducing a predetermined factor into a somatic cell, and exhibits excellent growth ability and pluripotency, and therefore is expected to be applied to regenerative medicine. In recent years, three-dimensional (3D) tissues (organoids) similar to living organs have been artificially produced and used using iPS cells. Organoids are generated by self-aggregation and self-assembly of stem cells, and imitate the process of occurrence of living organs. Since the structure and function of organoids are similar to those of living organs, organoids are also applied to basic studies such as development and physiology, elucidation of pathological conditions, drug discovery studies, and transplantation for regenerative medicine. The inventors have developed a method for three-dimensionally culturing iPSC with a bioreactor, inducing osteogenic differentiation, and producing an osteoblast cell aggregate of human iPSC (PTL 1). Although spheroids (cell aggregates) and organoids derived from iPSCs are promising biomaterials, transplantation of undifferentiated living cells has a risk of tumor development. In addition, since the implanted material is a foreign substance for a living body, it is also necessary to control immunogenicity and local inflammation.

Recently, a new concept of regenerative medicine has been proposed in which cells are inactivated while maintaining tissue components of a tissue produced in vitro from stem cells (NPL 1). By using the method, tissue regeneration can be induced by storing the tissue in a "seed" state for a long period of time and transplanting the tissue to a patient, so that a necessary amount of tissue can be supplied when necessary. Further, by inactivating the undifferentiated iPSC, it is possible to reduce the potential risk of tumorigenesis and control inflammation. A bone substitute having excellent osteoconductive ability and osteoinductive ability may be able to be produced by using a cell derived from stem cells as a material and inactivating the cells, but the production has not yet been put into practical use.

### Citation List

### Patent Literature

PTL 1: WO2018/181960
PTL 2: WO2015/064705
PTL 3: WO2020/175592

### Non Patent Literature

NPL 1: S. Pigeot, et al., Advanced Materials, 2021, 33, 2103737, https://doi.org/10.1002/adma.202103737.
NPL 2: H. Egusa, et al., Stem Cells Dev, 2014, 23(18), 2156-69.
NPL 3: H. Egusa, et al., Plos One, 2010, 5(9): e12743. doi: 10.1371/journal.pone.0012743.

### Summary of Invention

### Technical Problem

The present inventors have developed a bone substitute which inactivates cells while maintaining the activity of promoting bone remodeling and which is excellent in osteoconductive ability and osteoinductive ability. For inactivation of the cells, methods such as irradiation, heat treatment, and drug treatment are used. However, since these methods may denature a protein, it is considered that a freeze-drying method, which is capable of inactivating the cells while maintaining the physiological activity and a structure of the protein, is good. Compared with a drying method in the related art, since the freeze-drying method dries the material under a low temperature condition, thermal denaturation and chemical change of the material are reduced, a deterioration rate of the product in a drying process is reduced, and long-term storage at room temperature is enabled. However, it is not possible to deny a possibility that, depending on freeze-drying conditions, a chemical change such as oxidation, deamidation, or hydrolysis, or aggregation due to a change in higher-order structure of a protein, is caused. Such chemical and physical changes in a production process may reduce the physiological activity of the bone substitute for the purpose of implantation into the living body, and may act as an antigen. The present inventors inactivate iPS cells that are induced to differentiate into osteoblasts by freeze-drying to prepare a bone regeneration agent (PTL 2). Specifically, an iPS cell aggregate induced to differentiate into osteoblasts is initially frozen overnight in a freezer at -80°C, and then freeze-dried by a method in which the temperature is fixed to -10°C on a cooling stage and a pressure is gradually lowered to 6 Pa to 20 Pa over one night, and a bone substitute is produced without performing secondary drying. However, since a shelf type freeze dryer capable of accurately controlling a freeze-drying temperature by a program and having excellent drying efficiency is not used, it cannot be said that temperature control, time control, and sample drying in each step are sufficient. By improving and controlling the production method, there is a possibility that a bone substitute having a higher osteoinductive ability, a higher inflammation reducing ability, and the like can be produced.

The bone tissue is a composite material formed of cells, organic substrates, and inorganic substrates. Various biological materials are used for the treatment of femoral defects, whereas these biological materials have certain limitations in osteoinductive ability and production efficiency. An object of the invention is to provide a bone substitute having high safety and having higher osteoconductive ability and osteoinductive ability. Another object of the invention is to provide a method for producing a bone substitute that can be stably supplied, has high production efficiency, and is produced at lower cost.

### Solution to Problem

The invention relates to a bone substitute and a method for producing a bone substitute.
(1) A method for producing a bone substitute derived from a stem cell, includes: a step of inducing differentiation of a stem cell into an osteoblast to form a cell aggregate; an initial freezing step; and a primary drying step of performing freeze-drying at a temperature of -80°C or higher and -10°C or lower.

It is revealed that the production of a bone substitute by accurately adjusting the temperature in the freeze-drying step leads to the performance thereof. In particular, it is found that, in the primary drying step, the performance of the bone substitute is improved when the temperature in the drying chamber is kept constant by controlling the shelf temperature.

(2) The method for producing a bone substitute according to claim 1, further includes: a secondary drying step.

In the related art, although the secondary drying step is not performed, it is possible to produce a bone substitute having good performance by performing desorption of unfrozen water in the secondary drying step.

### Brief Description of Drawings

[FIG. 1A] FIG. 1A is a diagram schematically showing a step of freeze-drying a cell aggregate produced from iPS cells.
[FIG. 1B] FIG. 1B is a diagram schematically showing a configuration of a shelf type freeze dryer to be used.
[FIG. 2A] FIG. 2A shows examination of initial freezing conditions (1). A freezing temperature and a time are examined.
[FIG. 2B] FIG. 2B shows examination of initial freezing conditions (2). A stabilizer is examined.
[FIG. 3A] FIG. 3A shows examination of primary drying conditions (1). FIG. 3A is a diagram showing an examination result of a drying shelf temperature condition during freeze-drying.
[FIG. 3B] FIG. 3B shows examination of primary drying conditions (2). FIG. 3B is a diagram showing an examination result of a drying shelf temperature condition during freeze-drying, including -80°C.
[FIG. 3C] FIG. 3C shows examination of primary drying conditions (3). FIG. 3C is a diagram showing the expression of osteogenic differentiation marker genes under drying shelf temperature conditions during freeze-drying.
[FIG. 3D] FIG. 3D shows examination of primary drying conditions (4). FIG. 3D is a diagram showing an examination result of freeze-drying time.
[FIG. 4] FIG. 4 is a diagram showing an examination result of a secondary drying time.
[FIG. 5A] FIG. 5A is a diagram showing an evaluation result of implantation into a rat femoral defect model. FIG. 5A shows the evaluation result of mouse iPS-bone graft material (miPS-BGM) subjected to primary drying at various temperatures. FIG. 5A shows a 3D micro-CT image (top) and a cross-sectional image of a midline of a defective portion (bottom). Arrows indicate edges of femoral defects. Scale bar: 1.0 mm
[FIG. 5B] FIG. 5B is a diagram showing a result of quantitative analysis of bone parameters using a 3D micro-CT image. An amount of bone (BV/TV) and a bone mineral content (BMC) are analyzed at a defective site (average value ± SD, n = 3 to 7).
[FIG. 6A] FIG. 6A shows a hematoxylin and eosin (HE) stained image (upper stage) and an immunohistochemically stained image with an anti-galectin-3 antibody (lower stage) of a femoral defective site at 3 weeks after implantation of miPS-BGM. Arrows indicate edges of the formed defective portion. Scale bar: 1.0 mm
[FIG. 6B] FIG. 6B shows an enlarged image of a dottedline frame portion of the HE stained image in the upper stage of FIG. 6A. FIG. 6B is an enlarged image of a dotted line portion of the immunohistochemically stained image in the lower stage of FIG. 6A. * indicates remaining miPS-BGM, and arrows indicate neutrophil. Scale bar: 100 µm. The insertion drawing is an enlarged view of a portion surrounded by a square in each drawing. Insertion drawing scale bar: 10 µm.
[FIG. 6C] FIG. 6C shows an area of the remaining miPS-BGM (average value ± SD, n = 12 to 14).
[FIG. 7] FIG. 7 shows an effect of miPS-BGM on macrophage activity. Images show microscopic images of macrophages cultured in a growth medium (GM) or the miPS-BGM added growth medium after one day; scale bar: 200 µm. The graph shows the expression of inflammation and anti-inflammation-related genes one day or three days after culturing macrophages in a growth medium or a growth medium in which miPS-BGM is suspended.
[FIG. 8A] FIG. 8A is a diagram showing a total amount of proteins released from miPS-BGM produced at primary drying temperatures of -40°C, -10°C, and 30°C.
[FIG. 8B] FIG. 8B is a fluorescence image of F-actin of human bone marrow-derived mesenchymal stem cells (hMSC) cultured in presence of miPS-BGM. A circle indicated by a dotted line indicates a position of the miPS-BGM. Scale bar: 100 µm.
[FIG. 8C] FIG. 8C shows number of cells of hMSC around the miPS-BGM (average value ± SD, n = 3).
[FIG. 9] FIG. 9 shows results of wound healing assay. FIG. 9 shows a phase contrast microscope image (upper stage, scale bar: 200 µm) of hMSC immediately after scratch formation (0 hour) and after 9 hours of culture, and a graph (lower stage) showing the number of migrating cells (average ± SD, n = 3) in a scratch area after 9 hours of culture.
[FIG. 10] FIG. 10 shows an evaluation of miPS-BGM by cytokine antibody array. The miPS-BGM produced at a primary drying temperature of -40°C and β-TCP as a control were compared. An X-axis represents a fluorescence signal intensity of 308 target proteins, and a Y-axis represents a fluorescence signal intensity ratio (miPS-BGM/β-TCP intensity).
[FIG. 11] FIG. 11 is a diagram showing a proteome analysis result of miPS-BGM. FIG. 11 is a Venn diagram showing the number of proteins commonly or specifically identified between a miPS cell aggregate before freeze-drying and miPS-BGM.
[FIG. 12A] FIG. 12A is a macro image of miPS-BGM with different primary drying temperatures.
[FIG. 12B] FIG. 12B is a diagram showing a result of a compression test of miPS-BGM having different primary drying temperatures.
[FIG. 12C] FIG. 12C is an SEM image of miPS-BGM with different primary drying temperatures.
[FIG. 12D] FIG. 12D is an SEM image of miPS-BGM produced using a cooling stage type freeze dryer.
[FIG. 12E] FIG. 12E is a diagram showing an elemental composition analysis result of miPS-BGM produced using a shelf type freeze dryer.
[FIG. 12F] FIG. 12F is a diagram showing the elemental composition analysis results of miPS-BGM produced using a cooling stage type freeze dryer and human non-decalcified freeze-dried bone.
[FIG. 12G] FIG. 12G is a diagram showing measurement results of an amount of calcium eluted from miPS-BGM having different primary drying temperatures.
[FIG. 13A] FIG. 13A is an XRD pattern of miPS-BGM with different primary drying temperatures.
[FIG. 13B] FIG. 13B shows TEM/STEM images of miPS-BGM with different primary drying temperatures, scale, left panel: 200 nm, and right panel: 100 nm.
[FIG. 13C] FIG. 13C is a diagram showing a length of a fibrous apatite of miPS-BGM measured from the TEM image.
[FIG. 14] FIG. 14 shows an evaluation of human iPS-BGM by cytokine antibody array. A human freeze-dried bone (FDBA) was compared as a control.
[FIG. 15] FIG. 15 is a diagram showing a result of analyzing osteogenic differentiation inducing ability by human iPS-BGM having different primary drying temperatures by ALP staining.
[FIG. 16] FIG. 16 is a diagram showing results of a wound healing assay. FIG. 16 shows a phase contrast microscope image (upper stage) of hMSC cultured for 6 hours after scratch formation and a graph (lower stage) showing the number of migrating cells in a scratch area after 6 hours.

### Description of Embodiments

A bone substitute refers to a material used for the purpose of regenerating bone other than autologous bone, and can be used interchangeably with an artificial bone, a bone substitute, a bone regeneration agent, and the like. The bone substitute according to the invention uses stem cells as raw materials and has osteoconductive ability and osteoinductive ability. The osteoconductive ability refers to a function in which, when a medical material such as an implant is implanted into a natural bone in a living body, the bone is formed along a surface of the material, and the material and the bone are bonded to each other to be integrated with each other, and the osteoinductive ability refers to an ability to promote differentiation into osteoblasts such as mesenchymal stem cells in a place where the bone is damaged or denatured due to disease or injury to form a new bone.

Stem cells used in the production of the bone substitute according to the invention are not particularly limited as long as the stem cells can be induced into osteoblasts, and examples thereof include pluripotent stem cells and somatic stem cells. Examples of pluripotent stem cells include induced pluripotent stem cells (iPS cells), embryonic germ cells (EG cells), and embryonic stem cells (ES cells). Examples of somatic stem cells include mesenchymal stem cells, skeletal stem cells, hematopoietic stem cells, and neural stem cells. However, since the somatic stem cells differentiate into osteoblasts, mesenchymal stem cells or skeletal stem cells is preferable.

As described above, even with freeze-drying, depending on conditions, a chemical change such as oxidation, deamidation, or hydrolysis, or aggregation due to a change in higher-order structure of a protein may be caused. Therefore, a setting of the conditions of a freeze-drying method leads to the production of a bone substitute having good performance. For production of products such as regenerative medicine used for treatment, it is necessary to guarantee the quality of the products, and it is necessary to optimize a freeze-drying step and guarantee a certain quality. Further, since the freeze-drying step requires a long drying time and consumes a large amount of electric power, it is extremely important to optimize a freeze drying cycle in order to improve production efficiency while maintaining high quality. As shown in the following examples, the present inventors complete the invention by performing a freeze-drying step, particularly optimization of primary drying conditions. By optimizing the primary drying conditions, the bone replaceability of a bone transplantation material derived from miPS cells (mouse iPS) and human hiPS cells (human iPSC) in living body can be improved.

Next, the invention will be described with reference to data. In the following experiment, iPS cells derived from mouse gingival fibroblasts and human iPS cell-derived osteoblasts are used, whereas any cells may be used as long as the cells are stem cells. In addition, it is needless to say that the bone substitute can be produced in the same manner regardless of animal species. The iPS cells are not limited to gingival fibroblasts, and cells established from various cells such as blood cells, skin fibroblasts, and dental pulp cells can be used.

### 1. Culture of iPS Cells

The induction of stem cells into bone cells and the production of cell aggregates by three-dimensional culture are not limited to the following methods, and can be performed by known methods. The following experiment was performed using iPS cells derived from mouse gingival fibroblasts (miPSC, NPLs 2 and 3) prepared by introducing Oct 3/4, Sox2, and Klf4 with retrovirus. A miPSC is cultured on an inactivated SNLP76.7-4 feeder cell (donated by Dr. Allan Bradley of the Stanger Institute, United Kingdom) in an ES medium (Dulbecco's modified Eagle medium (DMEM, Nacalai Tesque, Inc.), 15% FBS (Biosera), 2 mM L-glutamine (Fujifilm Wako Pure Chemical Corporation), 1×10⁻⁴ M nonessential amino acids (Thermo Fisher Scientific K.K.), 1×10⁻⁴ M 2-mercaptoethanol (Thermo Fisher Scientific K.K.), and penicillin (50 U)/streptomycin (50 µg/mL) (Fujifilm Wako Pure Chemical Corporation)). The miPSCs were subcultured every 5 to 6 days using a 0.25 w/v% trypsin-1 mM/L EDTA solution. An embryoid body of miPSC was formed in an ES medium for 2 days using a low-adhesive culture dish. Thereafter, a suspension culture was further performed for 2 days in an ES medium supplemented with 1 µM of all-trans retinoic acid (NPL 2).

The SNLP76.7-4 cells used as a feeder cell were cultured in a medium containing Dulbecco's Modified Eagle Medium (DMEM, glucose 4.5 g/L), 7% fetal bovine serum (Japan Bio Serum), 2 mM L-glutamine, and penicillin (50 U)/streptomycin (50 µg/mL). When the cells reach 90% confluency, the cells were treated with 12 µg/mL of mitomycin C (Fujifilm Wako Pure Chemical Corporation) at 37°C for 2.5 hours, whereby DNA replication is inhibited, and the cells are inactivated. Thereafter, the cells were washed twice with physiological saline (PBS) and used as feeder cells.

### 2. Cell Aggregate Formation

The embryoid body was cultured in a 70 mL flask in an osteoblast differentiation medium (α-modified medium (Nacalai Tesque), 15% FBS (Gibco), 0.01 µM dexamethasone (Merck), 10 mM β-glycerophosphate (Merck), 50 µg/mL ascorbic acid-2-phosphoric acid (Merck), and 1% antibioticantifungal agent solution (Thermo Fisher Scientific)), and osteoblast differentiation is induced. The flask was placed in a seesaw shaker and shake-cultured at 0.3 Hz for 30 days. The osteoblast differentiation medium was replaced every two days.

### 3. Freeze-Drying

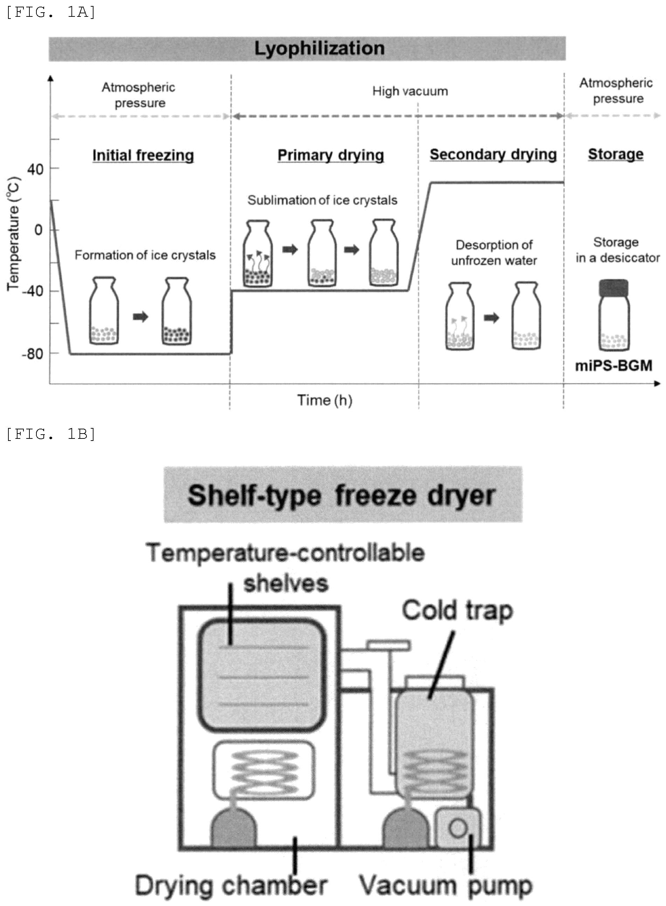

As described above, since the setting of the conditions of the freeze-drying method is considered to lead to the production of a bone substitute having good performance, the conditions of freeze-drying were examined in detail. The freeze-drying includes three stages of initial freezing (formation of ice crystals), primary drying (sublimation of ice crystals), and secondary drying (desorption of unfrozen water) (FIG. 1A). A typical shelf type freeze dryer includes a drying chamber, a condenser (cold trap), a decompression device (vacuum pump), and a temperature control device (FIG. 1B). In the shelf type freeze dryer, in order to cool and heat a product during a drying process, a shelf whose temperature can be controlled is disposed inside a drying chamber, and the temperature of the entire drying chamber is accurately controlled by controlling the temperature of a drying shelf in contact with a drying target, thereby achieving excellent drying efficiency. Since it is considered that accurate temperature control during drying is important, freeze drying is performed using a shelf type freeze dryer (EYELA DRC-1100, Tokyo Rikakikai Co., Ltd.), and any freeze dryer may be used as long as a sample temperature can be accurately controlled during drying. Examples of such a dryer include a sealed tube type freeze-drying system and a radiation heating type vacuum freeze-drying device. Since a shelf temperature adjustment range of the dryer is from -40°C to 30°C, a liquid nitrogen type vacuum freeze dryer (NRL-BC02S, Taiyo Nippon Sanso Corporation) is used only for the examination in which the primary drying temperature is set to -80°C.

After osteoblast differentiation induction for 30 days, a miPS cell aggregate was washed with PBS, transferred to a glass vial (diameter: 19 mm, length: 48 mm, Nippon Electric Glass Co., Ltd.), stored in a freezer at -80°C or -30°C until freeze-drying, and then rapidly transferred to a freeze-dryer to perform freeze-drying. The conditions of freeze-drying examined are summarized in Table 1. A drying shelf temperature was set to increase at a rate of 1.5°C/min between a primary drying step and a secondary drying step. After the secondary drying, the inside of the chamber was replaced with nitrogen gas in order to prevent deterioration of the freeze-dried miPS-BGM (miPS-bone graft material, and hereinafter, the freeze-dried miPS is referred to as miPS-BGM). The miPS-BGM was stored in an auto-dry desiccator (AS ONE Corporation) at room temperature (23°C) and a humidity of 15% or less.

**[Table 1]**

| A | | | | | |
|---|---|---|---|---|---|
| Initial freezing | | Primary drying | | Secondary drying | |
| temperature (°C) | time (h) | | | | |
| -30 | 1 | -10 (°C)/1h | | 20 (°C)/2h | |
| | 6 | | | | |
| | 12 | | | | |
| | 24 | | | | |
| -80 | 1 | | | | |
| | 6 | | | | |
| | 12 | | | | |
| | 24 | | | | |

| B | | | | | |
|---|---|---|---|---|---|
| Initial freezing | Primary drying | | | Secondary drying | |
| | shelf temperature (°C) | | time(h) | | |
| -80(°C)/24h | -80 | | 12 | 30°C/12h | |
| | -40 | | | | |
| | -30 | | | | |
| | -20 | | | | |
| | -10 | | | | |
| | 30 | | | | |

| C | | | | | |
|---|---|---|---|---|---|
| Initial freezing | Primary drying | | | Secondary drying | |
| | shelf temperature (°C) | | time (h) | | |
| -80(°C)/24h | -40 | | 0.5 | 30(°C)/2h | |
| | | | 1 | | |
| | | | 3 | | |
| | | | 6 | | |
| | | | 12 | | |

| D | | | | | |
|---|---|---|---|---|---|
| Initial freezing | Primary drying | | Secondary drying | | |
| | | | shelf temperature (°C) | | time (h) |
| -80(°C)/24h | -40°C/1h | | 30 | | 0.5 |
| | | | | | 1 |
| | | | | | 2 |

| E | | | | | |
|---|---|---|---|---|---|
| Initial freezing | Primary drying | | | Secondary drying | |
| | shelf temperature(°C) | | time (h) | | |
| -80(°C)/24h | -40 | | 24 | 30°C/6h | |
| | -10 | | | | |
| | 30 | | | | |

### 3.1 Examination of Initial Freezing Conditions

The initial freezing is a step of cooling the entire sample to a temperature equal to or lower than a eutectic point at which the sample is frozen. The step is extremely important in order to ensure porosity of the dried sample by forming and growing ice crystals. This is because if the freezing is insufficient, in the subsequent primary drying step, moisture rapidly expands as a degree of vacuum increases, which may lead to boiling of the sample. Since the conditions of the initial freezing step affect the efficiency of the subsequent drying step, the conditions were examined. Further, it was examined whether the use of various stabilizers such as saccharides and sugar alcohols in the initial freezing step can reduce damage to proteins and maintain stability during freezing and primary drying.

An extract of a miPS-BGM prepared under each condition in Table 1A is added to an osteoblast differentiation medium, and human bone marrow-derived mesenchymal stem cells (hMSC, donated by Dr. Mabuchi, Tokyo Medical and Dental University, and Dr. Matsuzaki, Shimane University) were cultured using the extract, and an osteoinductive ability of the miPS-BGM is evaluated by a degree of the differentiation. The hMSC was cultured in an MSC growth medium (DMEM (without pyruvic acid, 4.5 g/L glucose, Nacalai Tesque), 20% FBS (GE Healthcare), 1% penicillin (50 U)/streptomycin (50 µg/mL), 10 mM HEPES (Dojindo Laboratories), and 10 ng/mL of a recombinant b-FGF (Fujifilm Wako Pure Chemical Corporation)). The hMSC was cultured in a 10 cm culture dish at a cell density of 3×10⁴ cells/mL, 37°C, and 5% CO₂. The growth medium was replaced every 3 to 4 days. When the hMSC reached 80% confluency, cells were collected by trypsin and seeded in a 24-well plate at a cell density of 4×10⁵ cells/mL, and after 24 hours, culture was performed by replacing the growth medium with an osteoblast differentiation medium (Lonza).

The miPS-BGM was suspended in an osteoblast differentiation medium at a concentration of 5 mg/mL, and pulverized on ice with an ultrasonic pulverizer (BRANSON Digital Sonifier SFX150, EMERSON) at an ultrasonic amplitude of 20% for 2 minutes. After the ultrasonic treatment, the suspension was filtered through a syringe filter (pore size: 0.22 µm, Merck) and added to the hMSC osteoblast differentiation medium. The osteoblast differentiation medium was replaced every three days. After 7 days from the start of osteogenic differentiation induction, the hMSC is fixed with a 10% neutral buffered formalin solution (Fujifilm Wako Pure Chemical Corporation), calcium deposits were stained with alizarin red S (ARS), and a degree of osteogenic differentiation is evaluated.

The alizarin red S staining is performed as follows. After the fixed hMSC was washed twice with a deionized water, a 1% ARS solution (pH 6.3, Muto Chemical Co., Ltd.) is added, and the fixed hMSC was stained for 20 minutes at a room temperature with gentle shaking. After washing with deionized water four times, the hMSC was observed with an optical microscope (BX51, Olympus). For quantification of staining, 10% acetic acid (FUJIFILM Wako Pure Chemical Corporation) was added to each well, the plate was allowed to stand at room temperature for 30 minutes, and then the solution was collected in a microtube and stirred by a vortex mixer for 30 seconds. The sample was heated at 85°C for 10 minutes and then cooled on ice for 5 minutes. After centrifugation at 20,000 × g for 15 minutes, 10% aqueous ammonium hydroxide (Fujifilm Wako Pure Chemical Corporation) was added to a supernatant, and an absorbance of the supernatant was measured at 405 nm using a microplate reader (iMark, BIO RAD).

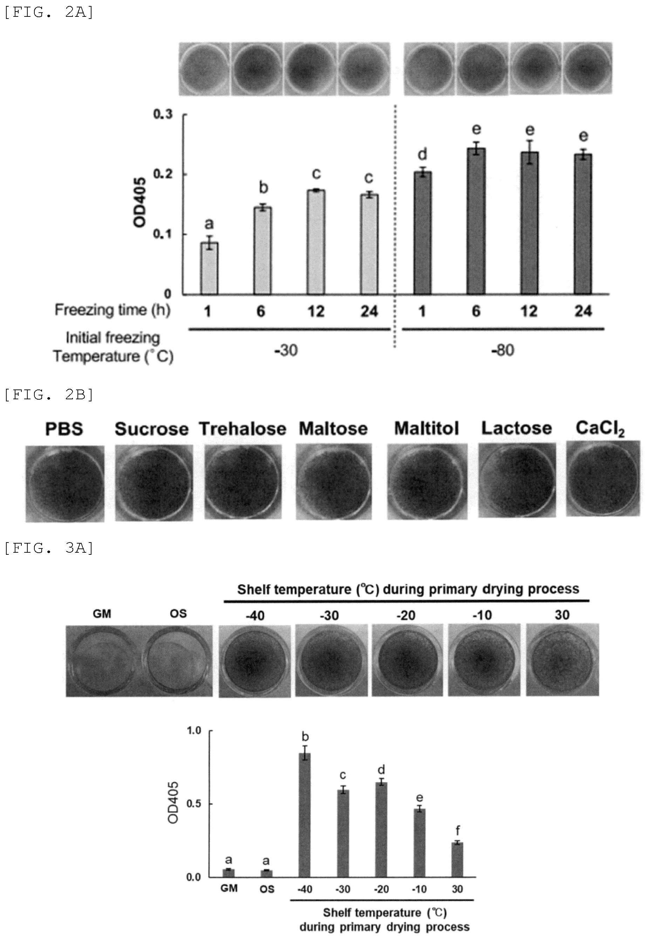

Results are shown in FIG. 2A. An upper stage shows images of the stained wells, and a lower stage shows quantitative results. The miPS-BGM subjected to the initial freezing at -80°C exhibited a higher osteogenic differentiation-promoting effect than the miPS-BGM subjected to the initial freezing at -30°C. The iPS-BGM prepared by initial freezing at -80°C for 6 hours or more significantly promoted osteogenic differentiation in the hMSC (P < 0.05). When the initial freezing is performed at -80°C, even for 1 hour, a higher osteogenic differentiation-promoting effect is observed than that observed after 24 hours at -30°C. Since no difference in the effect is recognized when the initial freezing is performed for 6 hours or more, a bone substitute having high osteogenic differentiation ability can be obtained by freezing for 1 hour or more, preferably 2 hours or more, more preferably 4 hours or more, and still more preferably 6 hours or more. In addition, regarding the initial freezing temperature, it is considered from these results that the freezing may be performed at a temperature of -40°C or lower, and preferably -60°C or lower.

Stabilizers such as saccharides and sugar alcohols are known to protect proteins when frozen, and therefore various stabilizers were examined. Sucrose (Merck), trehalose (Nacalai Tesque), maltose (Nacalai Tesque), maltitol (Nacalai Tesque), lactose (Nacalai Tesque), and CaCl₂ (Nacalai Tesque) were used as the stabilizers. Each of the additives was dissolved in PBS to obtain a 10% solution. A miPSC cell aggregate for one 25 cm² culture flask was allowed to stand in a PBS solution containing 5 mL of each additive at 4°C for 1 hour, and then was frozen at -80°C for 6 hours. The frozen sample was freeze-dried at room temperature (23°C) for 6 hours. The evaluation was performed in the same manner as described above with respect to promotion of osteogenic differentiation ability of the hMSC. Results are shown in FIG. 2B. None of the stabilizers changed the osteogenic differentiation ability of the miPS-BGM (FIG. 2B).

### 3.2 Examination of Primary Drying Conditions

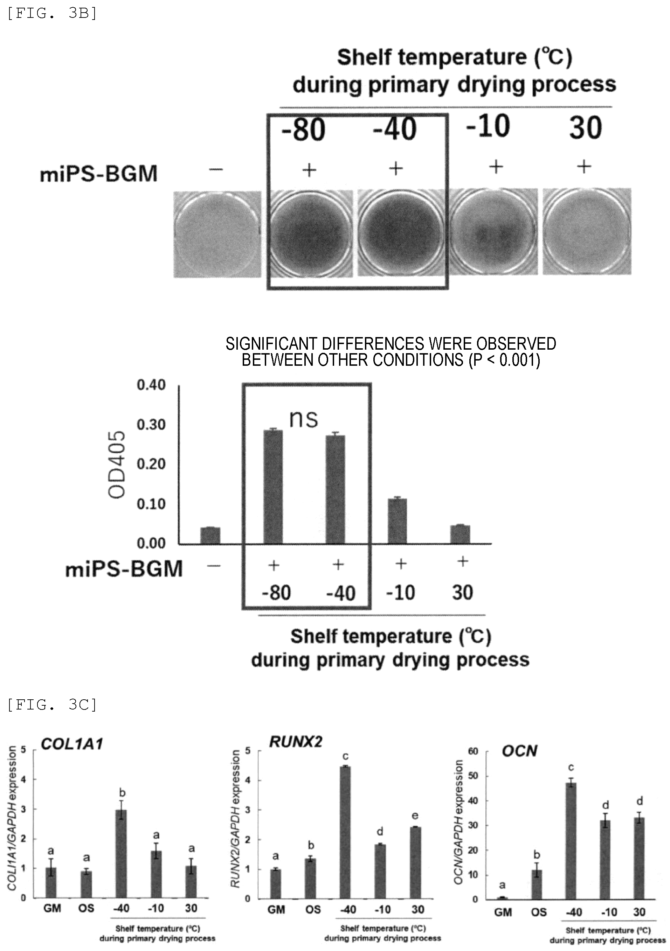

The temperature conditions of the primary drying were examined. As shown in Table 1B, the initial freezing was performed at -80°C, and the cell aggregates were primarily freeze-dried at different temperatures. Alizarin red S staining was performed in the same manner as described above, and the osteogenic differentiation inducing ability was evaluated. Results are shown in FIG. 3A. An upper stage shows an Alizarin Red S staining image of the hMSC on day 7 of osteogenic differentiation induction. In the drawing, GM denotes a growth medium, and OS denotes an osteoblast differentiation medium containing no miPS-BGM. A lower stage shows the quantitative results, and different letters indicate a significant difference (P < 0.05, according to Tukey's multiple comparison test following ANOVA. Unless otherwise specified, the same indication is used in the following experiments) (average value ± SD, n = 3). It is shown that the bone substitute prepared at a primary drying shelf temperature of -10°C had a significantly higher osteogenic differentiation inducing ability than the bone substitute prepared at 30°C. Further, when it is produced at a lower temperature of -20°C or - 40°C, it has higher osteogenic differentiation inducing ability. An analysis was performed as to whether a freeze-drying performed at a temperature lower than -40°C resulted in a higher osteogenic differentiation inducing ability (FIG. 3B). A miPS-BGM was prepared under a temperature condition of a primary drying temperature of -80°C described in Table 1B, and the osteogenic differentiation inducing ability was evaluated by alizarin red S staining. No difference was observed in the osteogenic differentiation inducing ability between the case in which the primary drying was performed at -80°C and the case in which the primary drying was performed at -40°C. Although no difference in performance is observed, setting the primary drying temperature to a lower value requires cooling costs, such as use of a liquid nitrogen. Therefore, performing the primary drying at -40°C is preferable from a cost perspective. Since, at -10°C, a higher osteogenic differentiation inducing ability is exhibited compared to 30°C (room temperature), the primary drying temperature may be set to -10°C or lower. Further, since a higher osteogenic differentiation inducing ability is exhibited at -20°C or lower, it is more preferable to perform the primary drying at -20°C or lower.

Further, expression of osteogenic differentiationrelated genes (COL1A1, RUNX2, and OCN) under each temperature condition was analyzed by a quantitative real-time RT-PCR method. After 7 days of culture of the hMSC in the osteogenic differentiation medium to which miPS-BGM was added, RNA was extracted from the cells using a TRIzol reagent (Thermo Fisher Scientific). RNA was isolated and purified using RNeasy Mini Kit (Qiagen). After DNA removal using a DNA-free DNA Removal Kit (Thermo Fisher Scientific), cDNA was synthesized from 1 µg of total RNA by a known method. The quantitative real-time RT-PCR analysis was performed using a Thunderbird SYBR qPCR Mix (Toyobo) and a StepOnePlus real-time PCR system (Thermo Fisher Scientific). The expression of a target gene was quantitatively analyzed by the 2-^{△△Ct} method and normalized to the expression of GAPDH. The primer sequences used are shown in Table 2. The primers used were primers for human sequences.

**[Table 2]**

| *G*ene name | Primer sequence (Fw, forward; Rv, reverse) | SEQ ID NO |
|---|---|---|
| *GAPDH* | Fw:5'-GAAGGTGAAGGTCGGAGTCA-3' | 1 |
| | Rv:5'-GAAGATGGTGATGGGATTTC-3' | 2 |
| *RUNX2* | Fw:5'-CAGACCAGCAGCACTCCATA-3' | 3 |
| | Rv:5'-CAGCGTCAACACCATCATTC-3' | 4 |
| *COL1A1* | Fw:5'-GTGCTAAAGGTGCCAATGGT-3' | 5 |
| | Rv:5'-CTCCTCGCTTTCCTTCCTCT-3' | 6 |
| *OCN* | Fw:5'-GGCAGCGAGGTAGTGAAGAG-3' | 7 |
| | Rv:5'-AGCAGAGCGACACCCTAGAC-3' | 8 |

The miPS-BGM freeze-dried at -40°C significantly increased the expression of the osteogenic differentiationrelated genes COL1A1, RUNX2, and OCN in the hMSC (P < 0.05) (FIG. 3C).

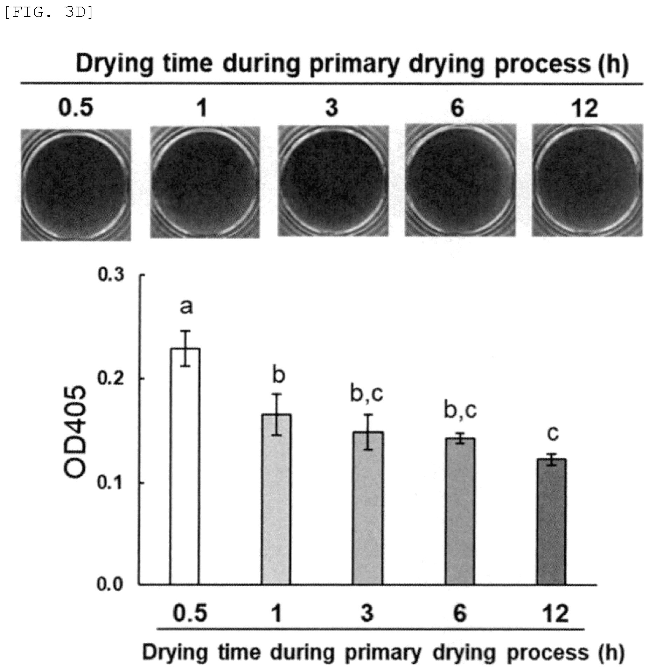

Next, the drying shelf temperature was fixed to -40°C, and the primary drying time was optimized. The initial freezing was performed at -80°C for 24 hours, and the secondary drying was performed at 30°C for 2 hours (Table 1C). As a result, it was revealed that, under the present condition, when the primary drying of the miPS-BGM was performed for 0.5 hours, the osteogenic differentiation of the hMSC was maintained highest (FIG. 3D). Since a time required for complete drying also varies depending on a volume of a sample and the like, a definitive statement cannot be made. However, if complete drying is achievable, a shorter drying time may be acceptable. For example, under these experimental conditions, the time may be 10 minutes or longer, and preferably 15 minutes or longer. Almost no difference was observed among the primary drying times of 1 hour, 3 hours, and 6 hours.

### 3.3 Examination of Secondary Drying Conditions

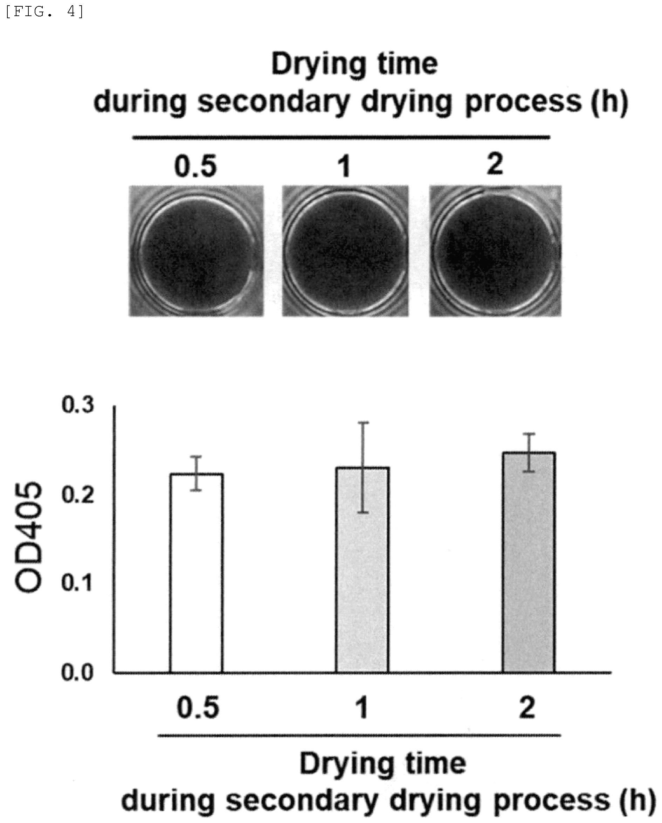

Next, the secondary drying time was optimized. Since the purpose of the secondary drying is to desorb unfrozen water, a drying temperature may be room temperature, and is 30°C here. The examination was conducted with the initial freezing set to -80°C for 24 hours and the primary drying time set to -40°C for 1 hour (Table 1D). As a result, it was revealed that osteogenic differentiation of the hMSC is maintained when the secondary drying time of the miPS-BGM is 0.5 hours or more (FIG. 4). Since unfrozen water is considered to be probably slight, it is considered that a shorter time, for example, about 10 minutes is sufficient.

### 4. Evaluation of iPS-BGM by Rat Femoral Defect Model

From the above experimental results, it is found that a shelf temperature during the primary drying greatly affects the quality of miPS-BGM. The conditions of the initial freezing and the secondary drying were set to be constant (initial freezing: -80°C/6 hours, secondary drying: 30°C/0.5 hours), and the conditions of the primary drying were changed (primary drying: -40°C, -10°C, or 30°C/0.5 hours).

A difference in quality of miPS-BGM due to a difference in temperature during primary drying was analyzed using a rat femoral defect model. A therapeutic effect of miPS-BGM prepared at different primary drying temperatures was evaluated using a rat model having a femoral defect. As the rat model, an 11-week-old Sprague-Dawley rat (CLEA) with a body weight of 340 f to 410 g (380 g on average) was used. Anesthesia was induced by inhalation of 2% isoflurane, and a combined anesthetic (1 mg/mL medetomidine hydrochloride (Domitor, Meiji Seika), 5 mg/mL midazolam (Dormicum, Astellas Pharma)) was used, and continuous anesthesia was carried out by adding 5 mg/mL butorphanol (Vetorphale, Meiji Seika Pharma Co., Ltd.) to 0.9% w/v sodium chloride (physiological saline, Otsuka Pharmaceutical). After shaving a left leg, the rat was disinfected with povidone-iodine (Kaneichi Chemical), and a local anesthesia was performed using xylocaine. The skin and muscles were incised to expose the femur, and a cortical femoral defect (3 mm × 5 mm, a critical size) was formed under perfusion using a round steel burr (Drendel+Zweiling Diamant) at the center of the femur. Thereafter, 0.2 cc of miPS-BGM was implanted in the defective portion of the femur. An implanted site was wrapped with a 10 mm × 20 mm absorbable collagen membrane (Koken Tissue Guide, Koken). The femur was splint-fixed using a prefabricated titanium stent, and then a central portion of the femur was wrapped and ligated with a water-cured polyurethane resincontaining glass fiber (Castlight α, Alcare) and a stainless steel wire. The muscles and skin were sutured using a 5-0 polypropylene suture (PROLENE, Ethicon). The rats were subjected to surgery on a heating mat at 37°C (Natsume Seisakusho Co., Ltd.). Three weeks later, the rats were euthanized, and a micro-CT and a histological examination were performed.

Three weeks after implanting the miPS-BGM, the femur defective portion was scanned using a micro-CT to quantify an amount of bone, a bone density, and a bone mineral content. A femur sample was fixed with 10% neutral buffered formalin at 4°C for 1 week. The amount of bone, the bone density, and the bone mineral content of the miPS-BGM implantation site were evaluated using a 3D micro X-ray CT apparatus (Scan Xmate-E090, Comscantechno) and bone structure analysis software (TRI/3D-BON, Ratoc System Engineering). X-ray scanning of the sample was performed at an energy level of 80 kV/60 µA through a brass filter having a thickness of 1 mm. A 3D image was reconstructed based on a calibration curve of the bone mineral content obtained by scanning the hydroxyapatite phantom under the same X-ray conditions. An analysis target region was set for each sample. A cortical region was defined as a region that connects, on both sides of a defect end, an outer basic lamella point and an inner basic lamella point with a straight line. A specific threshold value for the bone tissue was determined by superimposing the divided images on the original grayscale X-ray image.

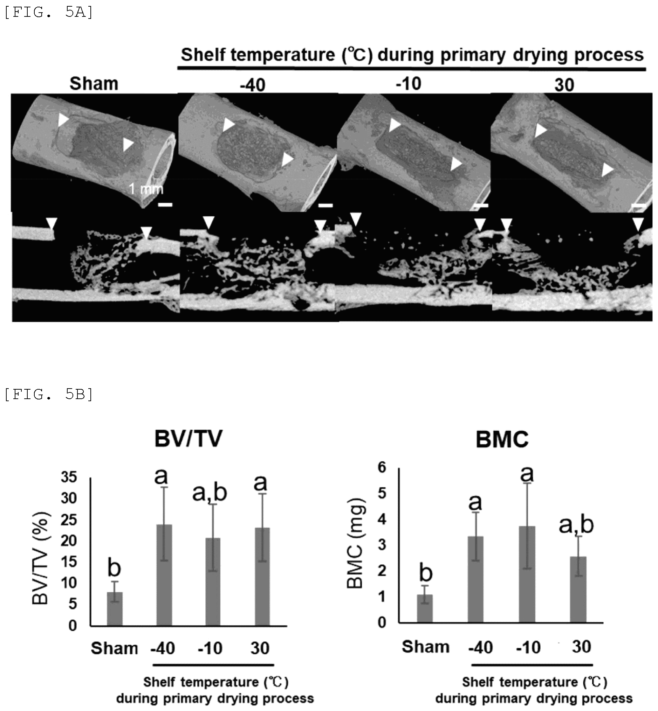

In a miPS-BGM implantation group, bone regeneration was promoted as compared with a non-implantation group (FIG. 5A). Further, when miPS-BGM was implanted, the formation of new bone was significantly higher than that in the non-implantation group (FIG. 5B).

Pathological analysis of femoral defects was performed. A femur sample was degreased by immersing the femur sample in 70% ethanol, followed by 100% ethanol, for one day each, for a total of two days, and then decalcified by placing the femur sample in a 0.5 mol/L EDTA solution (Fujifilm Wako Pure Chemical Corporation) for 4 weeks. After dehydration with ethanol, acetone, and xylene, paraffin embedding was performed. A paraffin section having a thickness of 3.5 µm was stained with hematoxylin and eosin (HE), and morphological observation was performed. A macrophage marker molecule, Galectin-3, was visualized by immunohistochemical staining. For the immunohistochemical staining, paraffin sections were deparaffinized by repeating twice a 5-minute washing with 100% xylene (Fujifilm Wako Pure Chemical Corporation) and with xylene/ethanol (50/50), and then rehydrated with graded ethanol (100%, 95%, 80%, 70%) and distilled water. Thereafter, an antigen was activated overnight at 60°C using a 10 mM citrate buffer (pH 6.0, Fujifilm Wako Pure Chemical Corporation). The slide was washed with PBS, then treated with 3% hydrogen peroxide (Fujifilm Wako Pure Chemical Corporation) at room temperature for 10 minutes to inhibit endogenous peroxidase activity. Thereafter, the slide was washed with PBS, and then allowed to stand for 30 minutes using 1% bovine serum albumin (Fujifilm Wako Pure Chemical Corporation) to stop the reaction. The slide was incubated overnight at 4°C with an anti-galectin-3 antibody (Galectin-3/LGALS3 Rabbit mAB, Cell Signaling Technology). Thereafter, the slide was washed with PBS containing Tween 20 (TBST), and then incubated with a secondary antibody (a mouse anti-rabbit IgG-HRP, Santa Cruz Biotechnology) at room temperature for 1 hour. The sample was washed with PBST, visualized by incubation with a DAB substrate (Merck) for 15 minutes, and then counter-stained with hematoxylin.

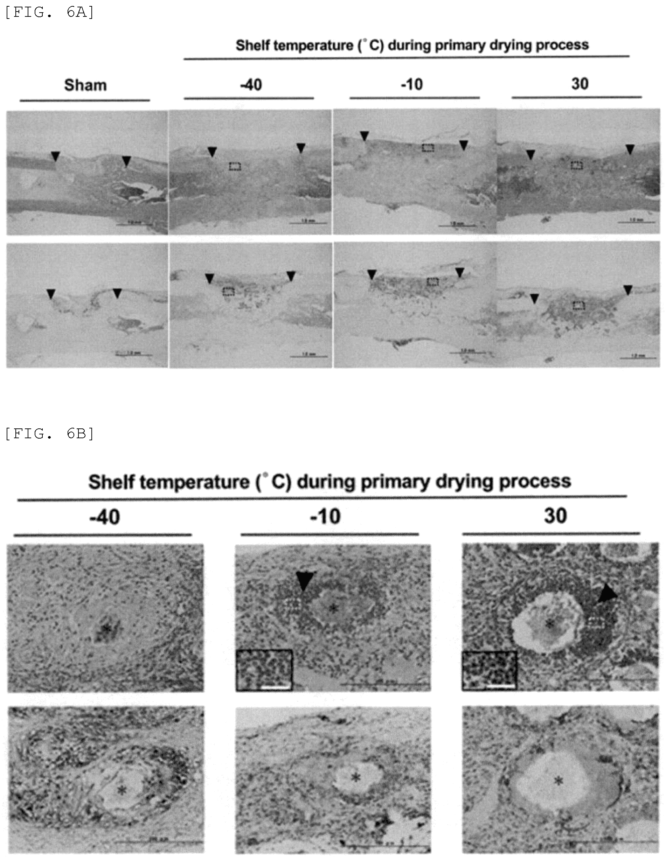

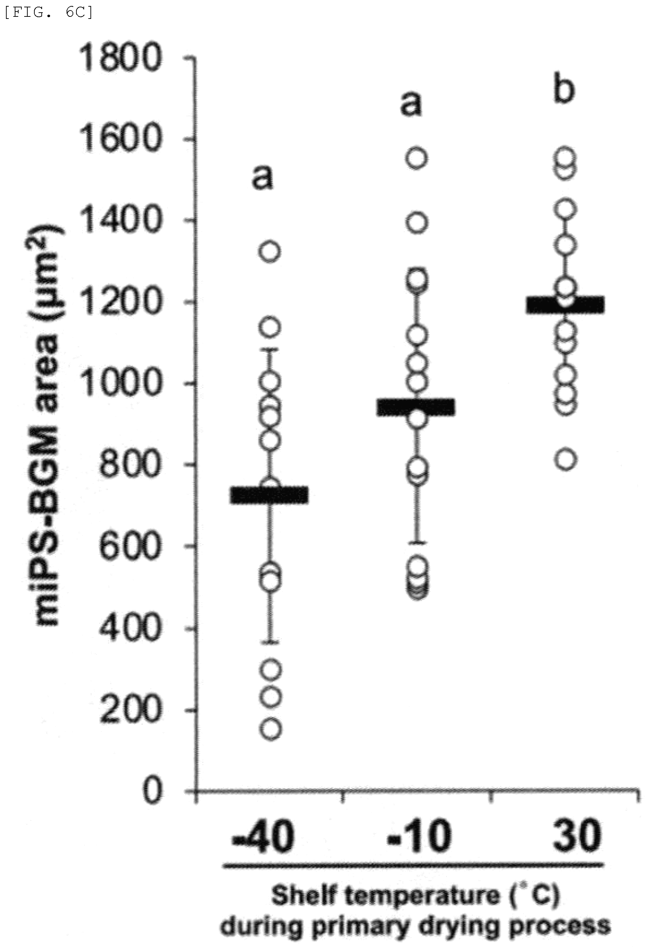

In HE staining of the bone defective portion, neutrophil infiltration, which is a marker of acute inflammation, was observed in the miPS-BGM implantation groups prepared at -10°C and at 30°C (hereinafter, a bone substitute is described by the primary drying temperature at which the miPS-BGM was prepared, such as miPS-BGM (-10°C) and miPS-BGM (30°C)) (upper stage of FIG. 6A and upper stage of FIG. 6B). On the other hand, galectin-3 positive macrophages accumulated around miPS-BGM (-40°C) (lower stage of FIG. 6A and lower stage of FIG. 6B), while few were observed around miPS-BGM (-10°C) and miPS-BGM (30°C). As a result of analyzing a total area of the miPS-BGM remaining in a granular form, the area of the miPS-BGM (30°C) was significantly larger than the area of the miPS-BGM (-40°C) and the area of the miPS-BGM (-10°C) (FIG. 6C). In the 17 rat femur samples implanted with the miPS-BGM, no tumor formation was observed either macroscopically or histologically.

### 5. Evaluation of Inflammatory Reaction

Effects of miPS-BGM prepared at various primary drying temperatures on inflammatory reactions were evaluated. A mouse macrophage-like cell line, J774A.1 (JCRB9108), was obtained from the Cell Bank of the National Institutes of Biomedical Innovation, Health and Nutrition. The cells were cultured under a humidified atmosphere at 37°C with 5% CO₂ using DMEM containing 4.5 g/L glucose without sodium pyruvate (Nacalai Tesque), 10% FBS (Japan Bio Serum), 2 mM L-glutamine (Fujifilm Wako Pure Chemical Corporation), and penicillin (50 U)/streptomycin (50 µg/mL). The cells were subcultured twice every three days while renewing the medium. After culturing to 80% confluency, the cells were collected and seeded on a 6-well plate at a cell density of 3.0×10⁵ cells/mL. After 24 hours, the medium was replaced with 5 mg/mL miPS-BGM suspension medium and cultured for 3 days, and the cells were collected and the expression of inflammatory genes and anti-inflammatory genes was analyzed by real-time RT-PCR. The primer sequences used are shown in Table 3. The primers used were primers for mouse sequences.

**[Table 3]**

| Gene name | Primer sequence (Fw, forward; Rv, reverse) | SEQ ID NO |
|---|---|---|
| *GAPDH* | Fw:5'-TGCACCACCAACTGCTTAG-3' | 9 |
| | Rv:5'-GGATGCAGGGATGATGTTC-3' | 10 |
| *II-10* | Fw:5'-GTTGCCAAGCCTTGTCAGAAA-3' | 11 |
| | Rv:5'-TTTCTGGGCCATGGTTCTCT-3' | 12 |
| *Tgfβ1* | Fw:5'-CTGGGCACCATCCATGAC-3' | 13 |
| | Rv:5'-CAGTTCTTCTCTGTGGAGCTGA-3' | 14 |
| *II-11* | Fw:5'-GCTGACAAGGCTTCGAGTAG-3' | 15 |
| | Rv:5'-TCTTTAGGGAAGGACCAGCT-3' | 16 |
| *II-1β* | Fw:5'-AAGGGCTGCTTCCAAACCTTTGAC-3' | 17 |
| | Rv:5'-ATACTGCCTGCCTGAAGCTCTTGT-3' | 18 |
| *Tnfα* | Fw:5'-CCACGCTCTTCTGTCTACTGAACT-3' | 19 |
| | Rv:5'-GGGTCTGGGCCATAGAACTG-3' | 20 |
| *II-6* | Fw:5'-CACTTCACAAGTCGGAGGCTTA-3' | 21 |
| | Rv:5'-GCAAGTGCATCATCGTTGTTC-3' | 22 |

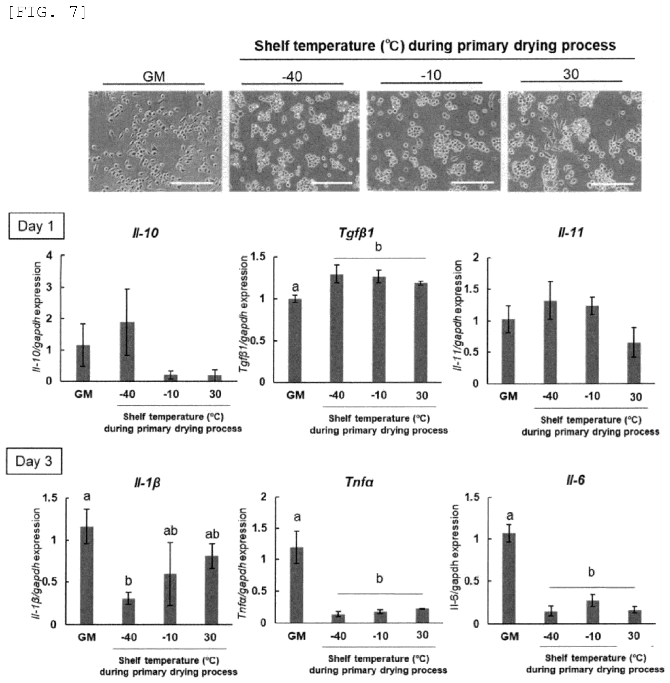

Macrophage J774A.1 cells were cultured in a growth medium containing a pulverized miPS-BGM, and gene expression of pro-inflammatory (Il-1β, Tnfα, Il-6) or anti-inflammatory (Il-10, Tgfβ1, Il-11) cytokines was analyzed using a real-time RT-PCR analysis on day 1 or day 3. As shown in the image in the upper stage of FIG. 7, in the group to which miPS-BGM produced at any temperature was added, the cells were collected and colonies were formed on day 1 of the addition, compared with a growth medium (GM) group. The gene expression of Tgfβ1 on day 1 of addition of miPS-BGM was significantly higher than that of the GM group. The expression of Tgfβ1, Il-10, and Il-11 genes showed higher values in the miPS-BGM (-40°C) addition group than in the GM group, the miPS-BGM (-10°C) addition group, and the miPS-BGM (30°C) addition group. The gene expression of Il-1β, Tnfα, and Il-6 was significantly decreased in any of the miPS-BGM addition groups as compared with the GM group. The gene expression of the Il-1β showed low values in the miPS-BGM (-40°C) addition group, although no significant difference was observed in the miPS-BGM (-40°C) addition group compared with the miPS-BGM (-10°C) addition group and the miPS-BGM (30°C) addition group (the lower stage graph of FIG. 7).

### 6. Protein Profile of miPS-BGM

### 6.1 Measurement of Amount of Protein

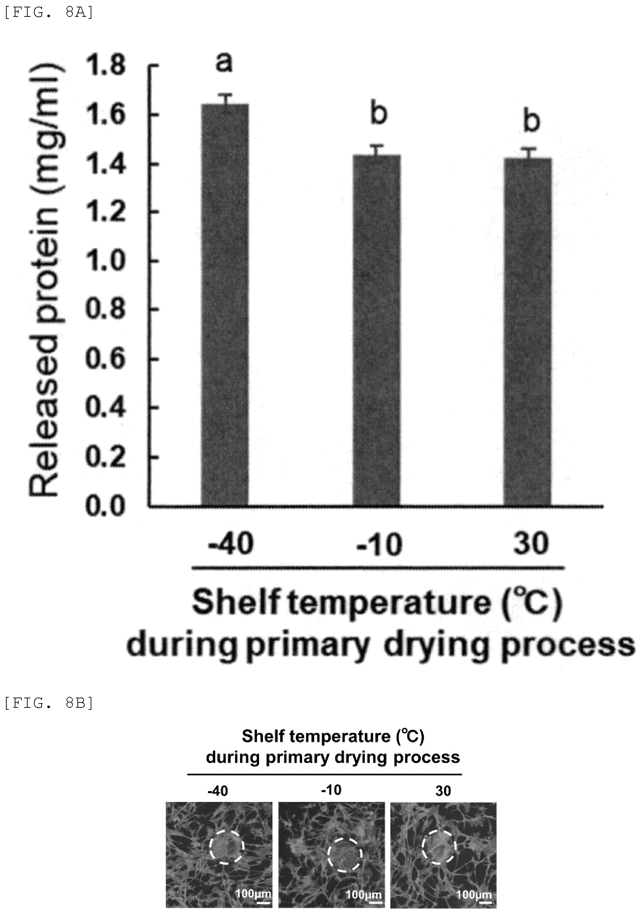

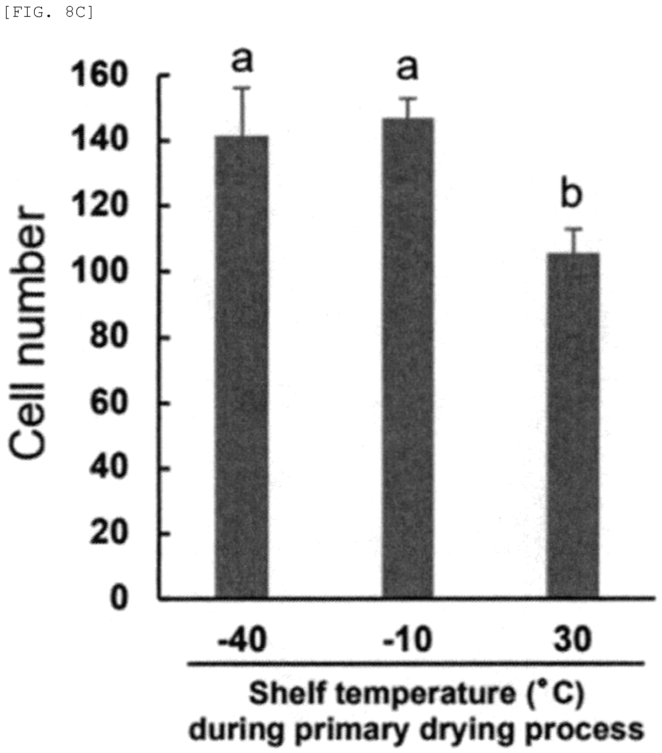

An amount of protein released from miPS-BGM produced at each primary drying temperature and suspended in PBS for 2 days was measured. 0.09 g of the miPS-BGM that was subjected to primary drying at each temperature was suspended in 500 µL of PBS, shaken at 37°C and 54 rpm, and after 2 days, a supernatant was collected. A concentration of the total protein released from the miPS-BGM was measured using a Pierce protein assay (Thermo Fisher Scientific). The miPS-BGM (-40°C) released significantly more protein than the miPS-BGM (-10°C) or the miPS-BGM (30°C) (FIG. 8A). In order to confirm an effect of a protein released from the miPS-BGM on proliferation and migration of the hMSC, the hMSC was cultured in the presence of the miPS-BGM that was subjected to primary drying at each of the temperatures of -40°C, -10°C, and 30°C. The hMSC and the miPS-BGM were mixed and cultured in a growth medium on a dish at a cell density of 5×10⁵. After 7 days, the hMSC was washed with PBS and then fixed at room temperature using 3.7% paraformaldehyde. The fixed cells were washed with PBS and subjected to permeation treatment using 0.5% Triton-X (FUJIFILM Wako Pure Chemical Corporation) at room temperature for 5 minutes. Thereafter, the cells were stained with Rhodamine Phalloidin (Thermo Fisher Scientific) for 40 minutes. After the cells were washed twice with PBS, the cells were reacted with a mounting medium containing DAPI (VECTASHIELD) for 20 minutes, and observed with a confocal laser scanning microscope (LSM780, Zeiss) (FIG. 8B). Immunofluorescence images of the miPS-BGM and the hMSC showed that the hMSC was accumulated around the miPS-BGM. As a result of quantifying the number of the hMSC, the number of cells around the miPS-BGM (-40°C) and the miPS-BGM (-10°C) was significantly increased compared to the miPS-BGM (30°C) (FIG. 8C).

### 6.2 Effect on Migration Ability

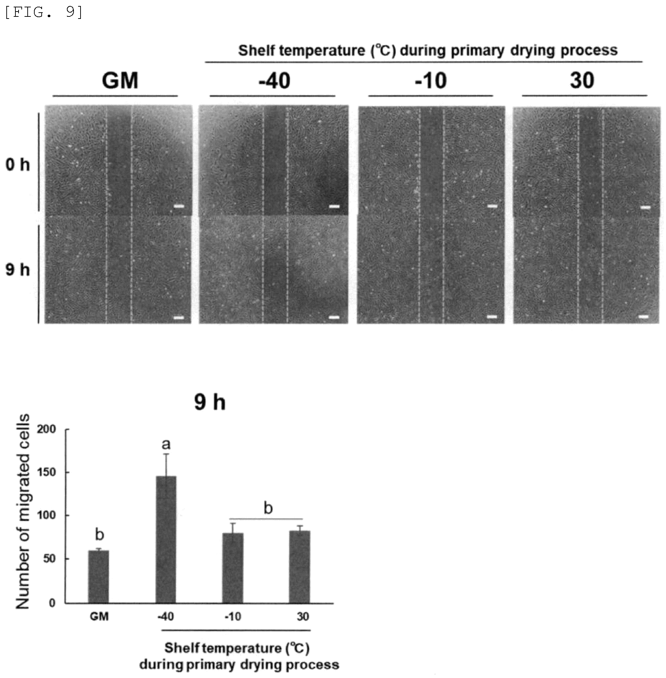

The influence of proteins released from the miPS-BGM on the cell migration ability of the hMSC was evaluated using a wound healing assay by scratching. The wound healing assay was performed as follows. The hMSC was seeded on a 12-well plate at a cell density of 1.0×10⁵ cells/mL. After 24 hours, the hMSC was linearly scraped off using 200 µL pipette tip to prepare a scratch. After gently washing the cell debris with the medium, the medium was replaced with a growth medium in which the miPS-BGM (-40°C), the miPS-BGM (-10°C), and the miPS-BGM (30°C) were respectively suspended at a concentration of 5 mg/mL. Immediately after the scratch and 9 hours later, an image of the hMSC was imaged and quantified using an Image J (NIH Image) (FIG. 9). The number of cells in a scratch area of each group was counted from the image. The number of migrating cells when the miPS-BGM (-40°C) was added to the medium was significantly larger than that when the miPS-BGM (-10°C) or the miPS-BGM (30°C) was added (P < 0.05) (FIG. 9, lower stage graph).

### 6.3 Analysis of Protein Components

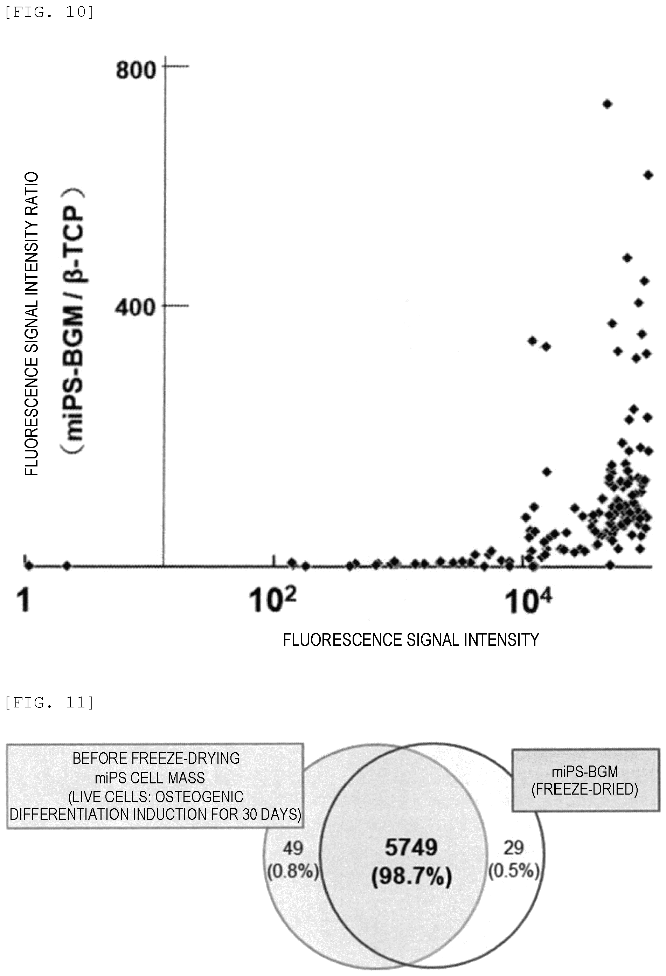

The above results indicate that a cell behavior around the miPS-BGM is mainly controlled by the constituent proteins of the miPS-BGM. Therefore, protein components of the miPS-BGM (-40°C) were comprehensively analyzed using a protein array (FIG. 10). After freeze-drying, 0.05 g of the miPS-BGM was added to 2 mL of PBS, and a mixture was pulverized with an ultrasonic pulverizer for 2 minutes. After centrifugation (1000 × g, 10 minutes), 1 mL of the supernatant was collected in a microtube and stored in a freezer at -80°C. As a control group, β-TCP (Kyocera), which is widely used as a bone substitute, was used. Using a RayBio (trademark) label-based mouse antibody array L-308 (RayBiotech), a total of 308 types of proteins in the miPS-BGM were evaluated. It was shown that the amount of 121 cytokines/cytokine-related proteins was larger compared with β-TCP. Although details are not shown here, in the miPS-BGM, a larger amount of the following proteins was maintained: bone metabolism-related proteins such as M-CSF, IL-27, adiponectin/Acrp30, and IL-6; osteoblast-related proteins such as SPARC, IL-17E, decorin, TGF-β1, osteoprotegerin, IGF-1, osteopontin, osteoactivin/GPNMB, and VCAM-1; and angiogenesis-related proteins such as PDGF C, endostatin, VEGF, and VEGFC.

In order to clarify the influence of the freeze-drying on the proteins constituting the miPS-BGM, a proteome analysis (mass spectrometry) was used to analyze a change in a protein composition before and after the freeze-drying. The miPS was subjected to osteogenic differentiation induction for 30 days, and a cell aggregate of 0.2 g was collected into a microtube, stored in a freezer at -80°C for 24 hours, and used as a cell aggregate before the freeze-drying. The cell aggregate before the freeze-drying and the miPS-BGM were evaluated by a DIA proteome analysis (LC-MS/MS (DIA), Promega) (FIG. 11). A total of 5812 proteins were identified, and 98.7% (5749 proteins) of these were common to both before and after the freeze-drying. It was revealed that the freeze-drying had almost no influence on the protein composition.

### 7. Formic Features of miPS-BGM

Morphological differences of the miPS-BGM prepared at different primary drying temperatures were evaluated using an appearance, a compression test, a scanning electron microscope (SEM), an energy dispersive X-ray analysis (EDX), a Ca concentration measurement, an X-ray diffraction (XRD), and a transmission electron microscope (TEM).

### 7.1 Feature of Form by Macro Observation

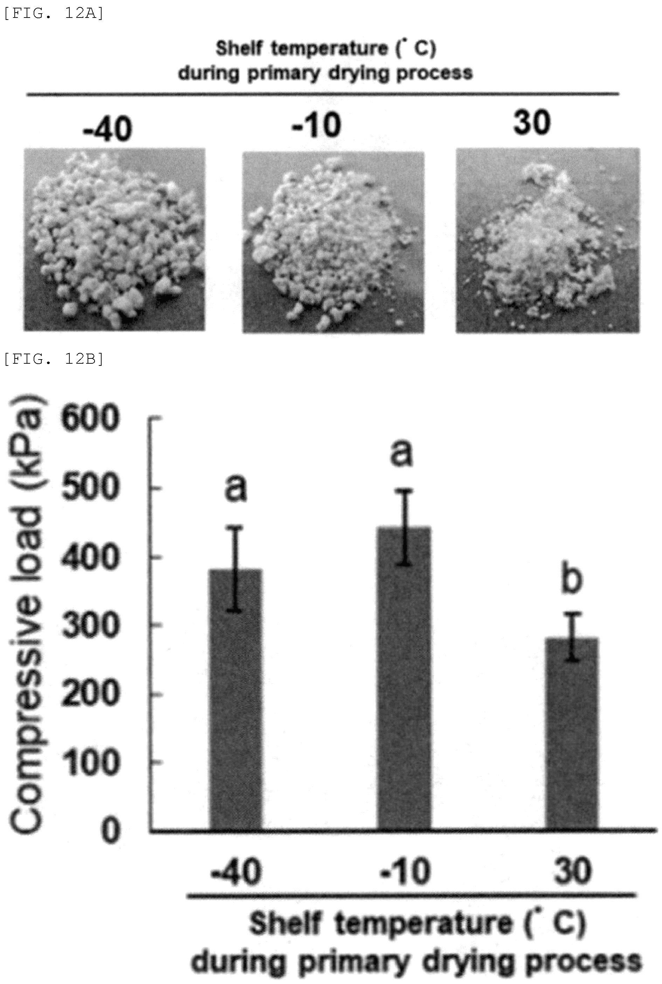

As shown in FIG. 12A, the morphology of the miPS-BGM (-40°C) and the miPS-BGM (-10°C) was round and granular, and diameters were also almost the same, whereas the miPS-BGM (30°C) did not maintain a granular morphology and exhibited a morphology as if finely pulverized.

### 7.2 Results of Compression Test

100 µL of miPSC cell aggregate was mixed with 1 mg of a gelatin hydrogel, and the mixture was filled into a polypropylene cylinder (4 mm in diameter, 4.5 mm in height) and frozen at -80°C for 24 hours to obtain a molded sample for a compression test. The freeze-drying was performed under the temperature conditions shown in Table 1E. A cylindrical sample was compressed and broken at a crosshead speed of 1.0 mm/min using an Instron universal testing machine (Instron 3342) equipped with a 50 N load cell. The operation of the testing machine and data analysis were performed using Bluehill (trademark) 2 software. All the tests were repeated six times, and an average value and a standard deviation were calculated. As a result of the compression test, a statistically significant increase in a compression load was observed for the miPS-BGM (-40°C) and the miPS-BGM (-10°C) compared to the miPS-BGM (30°C) (FIG. 12B, P < 0.05).

### 7.3 Observation with Scanning Electron Microscope

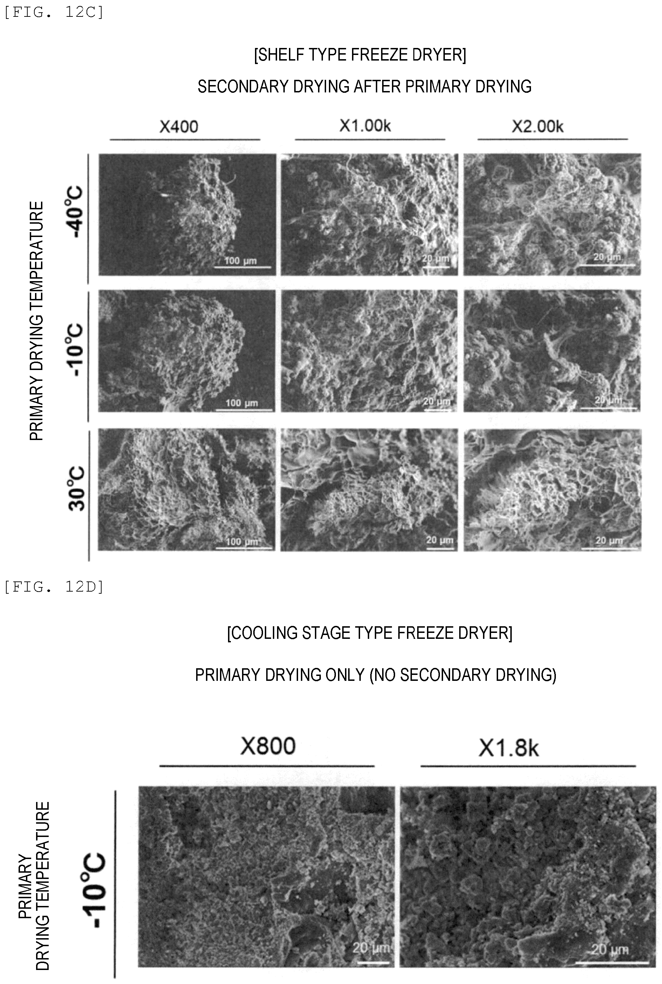

FIG. 12C shows a microstructure of the miPS-BGM prepared with a shelf type freeze dryer as observed by a SEM. The miPS-BGM that had been dried at each primary drying temperature and then subjected to secondary drying was observed with a scanning electron microscope (SU8000, Hitachi High-Tech Corporation) at an accelerating voltage of 5 kV. As a result of the SEM observation, in the samples of the miPS-BGM (-40°C) and the miPS-BGM (-10°C), an aggregate of spherical structures having a relatively uniform size with a particle diameter of about 10 µm (an average particle diameter of 7 µm) was observed. On the other hand, in the miPS-BGM (30°C), a flaky structure having an average particle diameter of 1 µm to 2 µm was observed.

FIG. 12D shows the microstructure of the miPS-BGM prepared by using a cooling stage type freeze dryer in the related art (JFD-320 Freeze Drying Device, JEOL Ltd.) with a primary drying temperature set to -10°C (PTL 2) as observed by an SEM. As a result of the SEM observation, an aggregate of spherical structures with a variation in particle diameter of 5 µm or less was observed, and compared with the miPS-BGM prepared at the same primary drying temperature using a shelf type freeze dryer capable of precisely controlling the freeze-drying temperature by a program (upper stage of FIG. 12C), a granular structure on a surface was smaller, and a shape inferior in porosity was observed. It is considered that the morphology by macroscopic observation and the granular structure by SEM observation correlate with the temperature during the primary drying. Therefore, a moisture content, porosity, and a true density closely related to these forms may correlate with the temperature during primary drying.

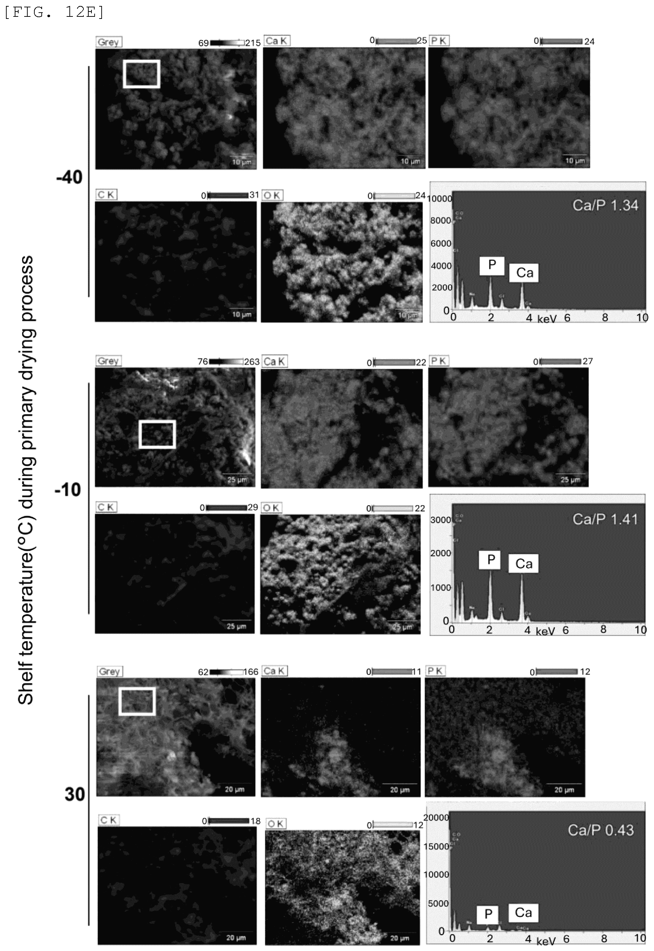

Further, the element composition of each miPS-BGM was evaluated by performing EDX on the miPS-BGM produced by the shelf type freeze dryer. From the SEM-EDX image, element distributions of Ca, P, C, and O were observed in a surface layer of the miPS-BGM, suggesting that carbonate apatite was contained. In addition, a Ca/P ratio (atomic composition%) was equal between the miPS-BGM (-40°C) and the miPS-BGM (-10°C), and was 1.34 and 1.41, respectively. On the other hand, the Ca/P ratio was 0.43 for the miPS-BGM (30°C) (FIG. 12E).

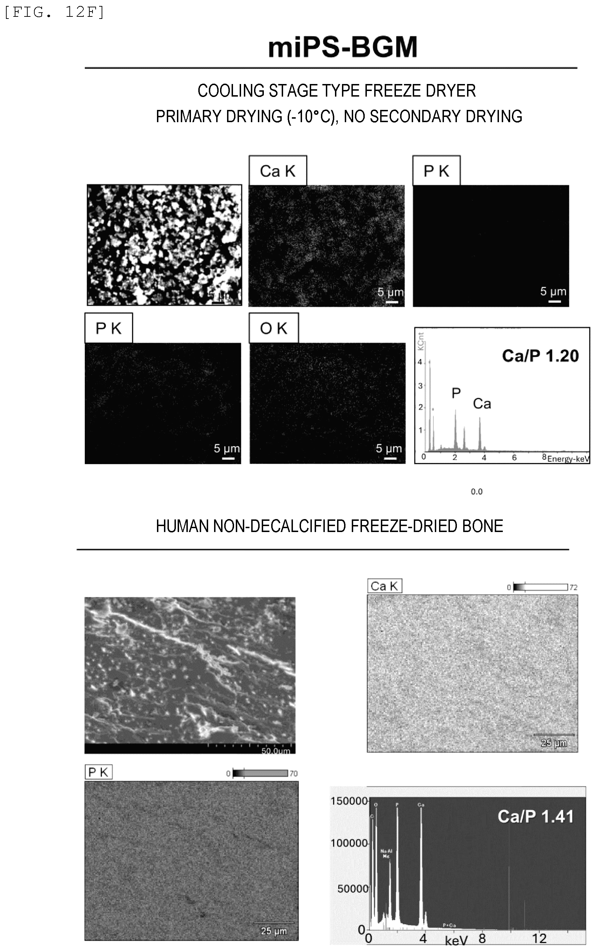

The EDX was performed on the miPS-BGM prepared by using a cooling stage type freeze dryer with a primary freeze-drying temperature set to -10°C (PTL 2) and on a human non-decalcified freeze-dried bone (OraGRAFT Cortical, LifeNet Health), and the Ca/P ratio (atomic composition%) was calculated (FIG. 12F). The Ca/P ratio of the miPS-BGM (PTL 2) was 1.20, which was lower than the Ca/P ratio (1.41) of the miPS-BGM (-10°C) produced by the shelf type freeze dryer described above. In addition, the Ca/P ratio of the human non-decalcified freeze-dried bone was 1.41, which was the same value as the Ca/P ratio of miPS-BGM (-10°C) produced by the shelf type freeze dryer. Therefore, it is suggested that the miPS-BGM produced by the shelf type freeze dryer has a Ca/P ratio closer to that of a natural bone as compared with the miPS-BGM of PTL 2, and is more suitable as an artificial bone (bone substitute).

Therefore, a reason why the properties of the miPS-BGM differ even if the temperature of the primary drying is apparently the same is considered to be due to the fact that, while a cooling stage type freeze dryer is able to perform freeze-drying by placing a sample on a cooled stage, the cooling stage type freeze dryer is unable to control a temperature of a drying chamber itself. The shelf type freeze dryer used this time can control the temperature in the drying chamber by controlling the shelf temperature, and since there is also a sensor in the chamber, the temperature can be more accurately controlled. Further, it is considered that this is because a vacuum state is rapidly reached 3 Pa or less in 10 minutes and drying can be rapidly performed. In order to produce a bone substitute having good performance, it is shown that temperature control during freeze-drying is important.

### 7.4 Calcium Elution Test

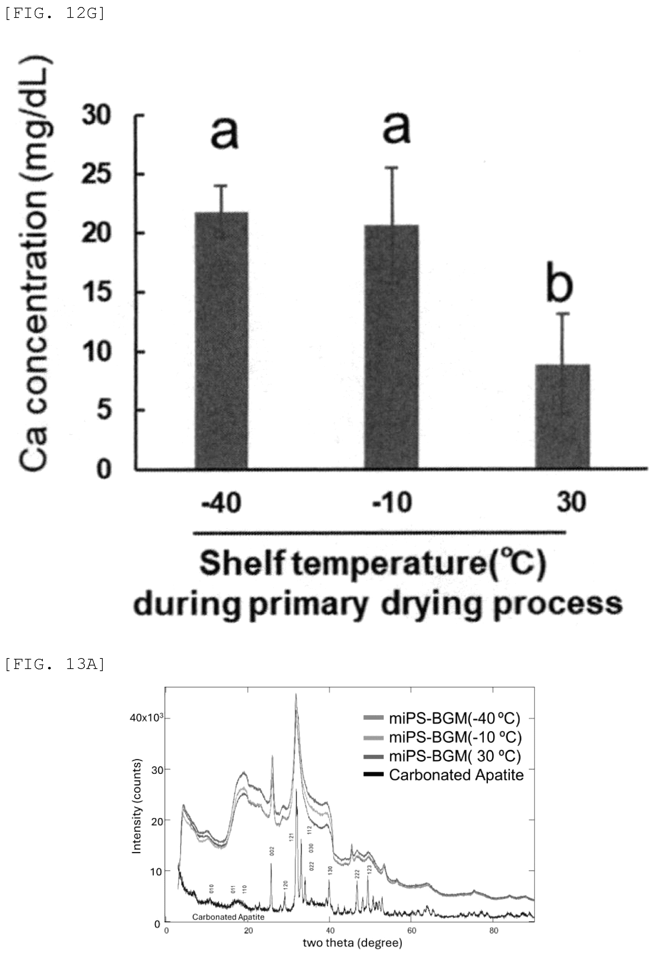

A calcium elution test of the miPS-BGM with different primary drying temperatures was performed. In the calcium elution test, the miPS-BGM was decalcified with 1.5 mL of 0.5 M acetic acid (FUJIFILM Wako Pure Chemical Corporation) and stored at room temperature (23°C) overnight. Insoluble components were removed by centrifugation at 2300 × g for 10 minutes. A calcium concentration of the supernatant was measured by the MXB method (OD595) using a calcium E-test kit (FUJIFILM Wako Pure Chemical Corporation). The calcium concentration of the miPS-BGM (30°C) was significantly lower than that of the miPS-BGM (-40°C) and that of the miPS-BGM (-10°C) (P < 0.05) (FIG. 12G).

### 7.5 Analysis by X-ray Diffraction (XRD)

Further, the microstructure was analyzed using X-ray diffraction (XRD). In the XRD, the composition of miPS-BGM was analyzed by X-ray diffraction analysis using a SmartLab 9SW (Rigaku Corporation). An XRD pattern is shown in FIG. 13A. The XRD pattern of the miPS-BGM was not affected by the temperature during primary drying. Characteristic peaks of hydroxyapatite at 2θ = 26.0 (002) and 2θ = 33.0 (112) corresponded to the pattern of carbonate apatite, and were high and sharp, indicating that the miPS-BGM has high crystallinity and purity.

### 7.6 Analysis by Transmission Electron Microscope (TEM)

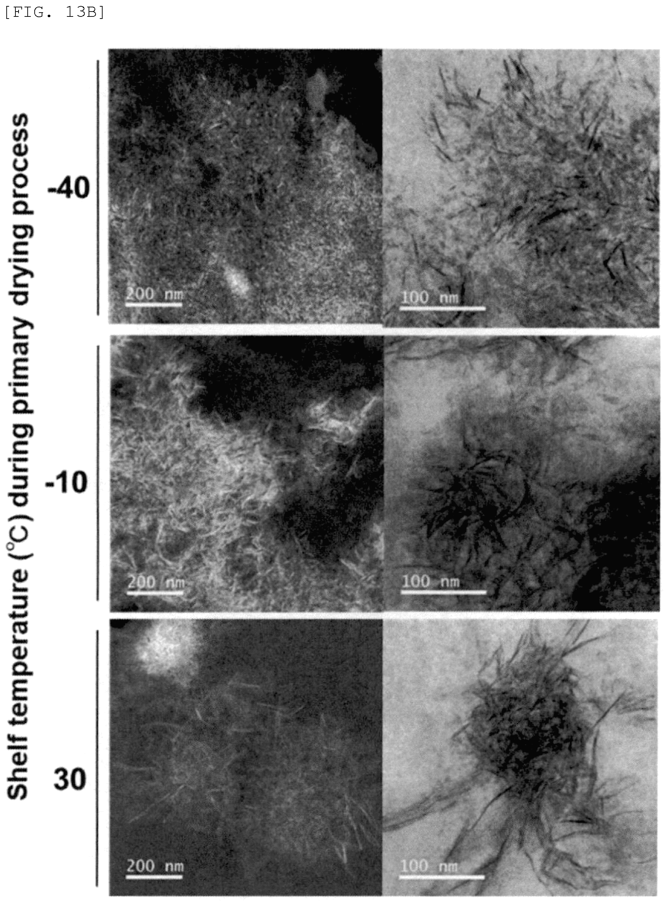

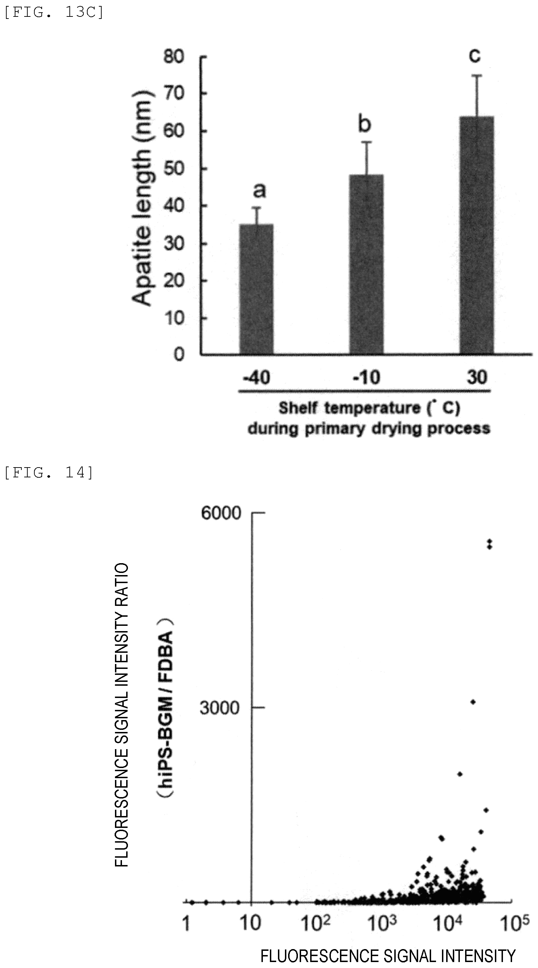

For the TEM observation, the miPS-BGM was embedded in a resin, an ultrathin section (0.05 µm in thickness) was prepared from a resin block, and the section was observed with a scanning TEM (JEM-ARM200F, JEOL Ltd.) at an accelerating voltage of 80 kV. From the TEM image of miPS-BGM, a cluster structure of needle-shaped particles was observed (FIG. 13B). A length of a needle-shaped structure of the miPS-BGM (-40°C) was 35 nm on average, which was significantly shorter than a length of the needle-shaped structure of the miPS-BGM (-10°C) (48 nm) and that of the miPS-BGM (30°C) (64 nm) (P < 0.05). The length of the needle-shaped structure was correlated with the temperature during the primary drying (FIG. 13C). Therefore, the iPS-BGM having different temperature conditions of the primary drying can be distinguished by an average length of the needle-shaped structure. Since there is a report that a short needle-shaped hydroxyapatite crystal exhibits an anti-inflammatory effect on an increase in expression of an anti-inflammatory factor such as IL10 of macrophages, it is suggested that a short needle-shaped crystal structure obtained under dry conditions at -40°C may act on local immune control.

### 8. Analysis of Human iPS Cell-Derived Bone Substitute

### 8.1 Analysis of Protein Components of Human iPS Cell-Derived Osteoblast Cell Aggregate

The protein components of the human iPS cell-derived osteoblast cell aggregates before freeze-drying were analyzed. Human iPS osteoblast cell aggregates were prepared according to PTLs 1 and 3. The human iPS osteoblast cell aggregates were collected into an Eppendorf tube and stored in a freezer at -80°C. As a comparison target, a human iPS cell aggregate before start of osteoblast differentiation induction was used. Protein expression and relative quantification analysis was performed by Kazusa Genome Technologies, Inc. using DIA proteome analysis (Promega K.K.).

From the mass spectrometry, 7678 types of proteins contained in the human iPS osteoblast cell aggregate were identified. As a result of removing proteins constantly expressed in human iPS cells (proteins expressed before osteoblast differentiation induction) and extracting those with a high expression level, it was revealed that the extracted proteins contain a large amount of proteins involved in bone metabolism, such as OGN, IGFBP5, CD34, TIMP1, FBLN5, LPAR1, IGFBP7, MGP, DCN, PDGFRB, IGF1, TAB2, FGFR2, and SPP1. Collagen, COL1A2, COL4A1, COL5A1, COL6A6, COL15A1, COL16A1, COL21A1, and COL26A1 were detected.

### 8.2 Analysis of Human iPS Cell-Derived Bone Substitute by Antibody Array

In the same manner as in 6.3, the protein components of a human iPS cell-derived bone substitute (hiPS-BGM) were also analyzed. The hiPS-BGM was prepared according to the method of the inventors (PTLs 1 and 3). After the hiPS-BGM was pulverized in PBS using an ultrasonic wave, a supernatant collected using a centrifuge was stored in a freezer at -80°C. As a comparison target, human freeze-dried bone (FDBA: OraGRAFT (LifeNet Health)) was treated in the same manner to prepare a supernatant sample. A comprehensive analysis using an antibody array was performed using Human Antibody Array L-1000 (Filgen, Inc.). The proteins contained in hiPS-BGM and FDBA were compared and analyzed, and compared with the protein data contained in miPS-BGM to examine common proteins (FIG. 14).

While an existing bone substitute (FDBA) contained almost no protein, the hiPS-BGM was revealed to contain a large amount of proteins related to bone tissue regeneration, such as bFGF, FGF-23, BMP-2, BMP-3, DMP-1, ALPP, GDF1, BMP-5, IGF-II, IGFBP-2, IGF-I, BMP-15, BMP-8, BMP-7, Osteoprotegerin/TNFRSF11B, and BMP-9; angiogenesis-related proteins such as EG-VEGF/PK1, Angiopoietin-like 1, Angiopoietin-like Factor, PDGF-AA, Angiopoietin-2, VEGF R3, VEGF R1, and VEGF; and proteins related to bone metabolism such as Beta IG-H3, IL-17B R, IFN-beta, Calcitonin, IL-17R, MMP-10, and TNF-beta. It is also revealed that the hiPS-BGM abundantly contains proteins such as COCO, Cadherin-13, Clusterin, C5/C5a, CD30 Ligand/TNFSF8, Kallikrein 5, IL-4, IGFBP-1, IL-1 F8/FIL1 eta, GRO, IL-8, Glut3, IL-29, ALCAM, CNTF, IL-21 R, CD163, IL-5, CD71, IL-21, EDG-1, Prolactin, IL-26, Growth Hormone (GH), GDF11, CD40 Ligand/TNFSF5/CD154, Cathepsin B, GDF8, GM-CSF, IL-7, CCR4, BNP, IL-2, IL-6, C-peptide, IL-17, GREMLIN, and BTC. As a result of comparing the hiPS-BGM data (paired with FDBA) with the miPS-BGM data (paired with FDBA), 68 types of common proteins were identified (Table 4).

**[Table 4]**

| | | | |
|---|---|---|---|
| IL-15 | VEGF | MFG-E8 | IL-17E |
| IL-1 alpha | SIGIRR | Crossveinless-2 | IL-20 R alpha |
| IL-17R | SLPI | Prolactin | Soggy-1 |
| CCR6 | Dkk-3 | IL-9 | VE-Cadherin |
| IL-6 | IGFBP-2 | IGF-II | IFN-beta |
| Cardiotrophin-1 | Frizzled-7 | IL-15 R alpha | IGFBP-1 |
| ICAM-1 | IGFBP-3 | SPARC | TGF-beta RII |
| SDF-1 | L-Selectin | Endostatin | IL-3 R alpha |
| DAN | Galectin-3 | IL-17 | CXCR6 |
| TGF-beta 1 | Fas Ligand | IL-10 | Decorin |
| Dtk | CXCL14/BRAK | IL-11 | IL-5 |
| CCL28 | IGF-I | EG-VEGF/PK1 | EDAR |
| CD14 | CTACK | CXCR2/IL-8 RB | EGF R |
| TNF-alpha | IL-20 | Glut2 | Artemin |
| PDGF R beta | CXCL16 | Adiponectin/Acrp30 | TNF RII |
| Osteoactivin/GPNMB | IGFBP-6 | MCP-1 | Fractalkine |
| CXCR3 | IL-2 R alpha | Fas/TNFRSF6 | TIMP 1 |

### 9. Evaluation of Osteoinductive Ability of hiPS-BGM

The temperature conditions for primary drying of hiPS-BGM were examined. In the same manner as for the miPS-BGM, the cell aggregate was initially frozen at -80°C, then subjected to primary freeze-drying at different shelf temperatures, and prepared. An extract of the hiPS-BGM was added to an osteogenic differentiation medium, and using the medium, human bone marrow-derived mesenchymal stem cells (hMSC) were cultured, and an osteoinductive ability of the hiPS-BGM was evaluated by a degree of the differentiation. The hMSC was induced by an osteogenic differentiation medium in the same manner as in the method described in 3.1.

The hiPS-BGM was suspended in an osteogenic differentiation medium at a concentration of 5 mg/mL and pulverized on ice with an ultrasonic pulverizer at an ultrasonic amplitude of 20% for 2 minutes. After the ultrasonic treatment, the suspension was filtered through a syringe filter (pore size: 0.22 µm, Merck) and added to the hMSC osteoblast differentiation medium. The osteoblast differentiation medium was replaced every three days. After 14 days from the start of osteogenic differentiation induction, the hMSC is fixed with a 10% neutral buffered formalin solution (Fujifilm Wako Pure Chemical Corporation), stained for alkaline phosphatase (ALP), and the degree of osteogenic differentiation is evaluated.

The ALP staining was performed as follows. After the fixed hMSC was washed three times with purified water, a chromogenic substrate (an ALP staining kit, Cosmo Bio Co., Ltd.) was added, and the fixed hMSC was stained at 37°C for 20 minutes. After washing with purified water three times, the hMSC was observed with an optical microscope (BX51, Olympus Corporation). For quantification of staining, the ALP staining positive range area was analyzed with ImageJ image analysis software.

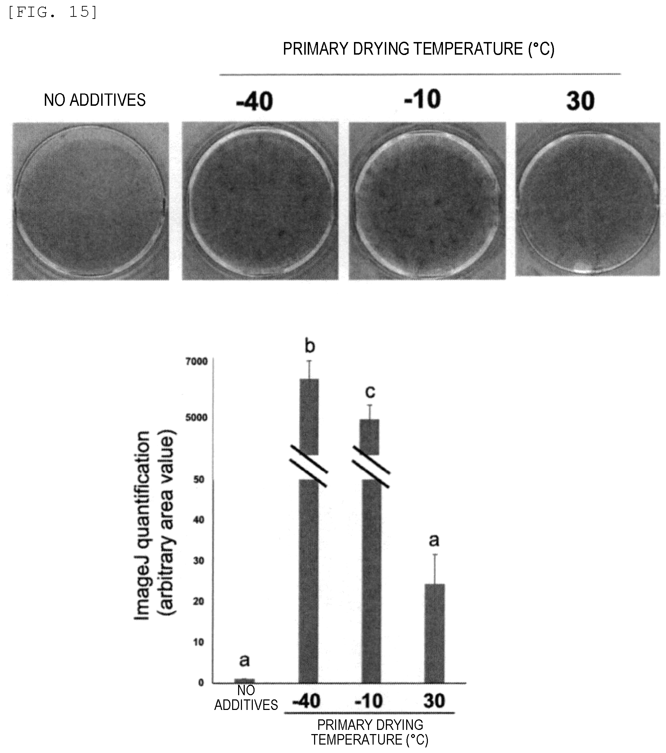

Results are shown in FIG. 15. An upper stage shows an ALP staining image on day 14 of osteogenic differentiation induction, and a lower stage shows quantitative results. In the drawing, no addition indicates an osteogenic differentiation medium that does not contain the hiPS-BGM. The lower stage shows the quantitative results (average value ± SD, n = 3). It is shown that the bone substitute prepared at a primary drying shelf temperature of -10°C had a significantly higher osteogenic differentiation inducing ability than the bone substitute prepared at 30°C. Further, when it is produced at a lower temperature of -40°C, it has higher osteogenic differentiation inducing ability. Since, at -10°C, a higher osteogenic differentiation inducing ability is exhibited compared to 30°C, the primary drying temperature may be set to -10°C or lower. Further, since a higher osteogenic differentiation inducing ability is exhibited at -40°C or lower, it is more preferable to perform the primary drying at -40°C or lower.

### 10. Effects of hiPS-BGM on Migration Ability

The influence of proteins released from the hiPS-BGM on the cell migration ability of the hMSC was evaluated using a wound healing assay by scratching. The wound healing assay was performed using the hMSC in the same manner as in 6.2. The hMSC was seeded on a 12-well plate at a cell density of 4.0×10⁵ cells/mL. After 24 hours, the hMSC was linearly scraped off using 200 µL pipette tip to prepare a scratch.

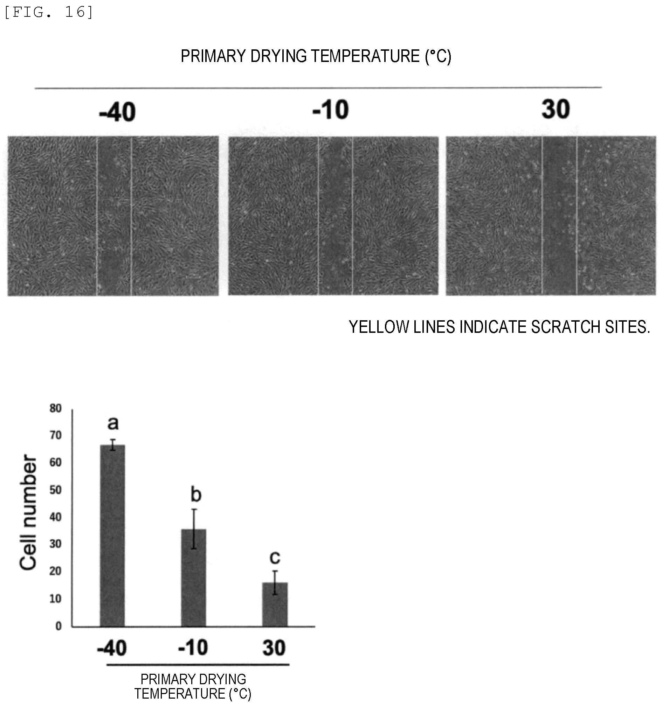

After gently washing the cell debris with the medium, the medium was replaced with a growth medium in which the hiPS-BGM (-40°C), the hiPS-BGM (-10°C), and the hiPS-BGM (30°C) were respectively suspended at a concentration of 5 mg/mL. The suspension was performed using an ultrasonic pulverizer according to the above-described method. Immediately after the scratch and after 6 hours, an image of the hMSC was captured (FIG. 16, the upper stage image shows an image after 6 hours), and the number of cells migrated in the scratch area was analyzed. The number of cells in a scratch area of each group was counted from the image. The number of migrating cells when the hiPS-BGM (-40°C) was added to the medium was significantly larger than that when the hiPS-BGM (-10°C) or the miPS-BGM (30°C) was added (P < 0.001) (FIG. 16, lower stage graph).

As described above, a bone substitute excellent in osteoconductive ability and osteoinductive ability can be produced by performing freeze-drying to inactivate cells without a risk of tumor formation and optimizing temperature and time. Optimal conditions were examined for all of the three steps of freeze-drying, initial freezing, primary drying, and secondary drying. In any step, the quality changes depending on a treatment temperature and time, whereas the conditions of the primary drying are particularly important, and it is necessary to accurately maintain the temperature of the sample. In terms of cost, it is preferable to perform the three steps in a shorter time and at a higher cooling temperature.

Here, the analysis was performed in detail using mouse iPS cells, and the analysis results of protein components, osteoinductive ability, and migration ability of human iPS cells were equivalent to the results obtained in mice. Therefore, the results obtained using mouse iPS cells can be directly applied to a method for producing a bone substitute using human iPS cells.

## Claims

1. A method for producing a bone substitute derived from a stem cell, comprising:
a step of inducing differentiation of a stem cell into an osteoblast to form a cell aggregate;
an initial freezing step; and
a primary drying step of performing freeze-drying at a temperature of -80°C or higher and -10°C or lower.

2. The method for producing a bone substitute according to claim 1, further comprising:
a secondary drying step.

3. The method for producing a bone substitute according to claim 1, wherein
the primary drying step is performed for 10 minutes or longer and 6 hours or shorter.

4. The method for producing a bone substitute according to claim 1, wherein
the initial freezing step is performed at a temperature of -40°C or lower and -80°C or higher.

5. The method for producing a bone substitute according to claim 4, wherein
the initial freezing step is performed for at least one hour.

6. A bone substitute derived from pluripotent stem cells produced by the production method according to any one of claims 1 to 5.
